(19) Europäisches Patentamt
European Patent Office
Office européen des brevets

(11) EP 1 511 852 B1

# (12) EUROPEAN PATENT SPECIFICATION

(45) Date of publication and mention of the grant of the patent:
**26.05.2010 Bulletin 2010/21**

(21) Application number: **03812412.9**

(22) Date of filing: **05.06.2003**

(51) Int Cl.:
***C12Q 1/00*** *(2006.01)* ***G01N 33/566*** *(2006.01)*
***G01N 35/00*** *(2006.01)* ***G01N 24/00*** *(2006.01)*

(86) International application number:
**PCT/US2003/017729**

(87) International publication number:
**WO 2004/051214 (17.06.2004 Gazette 2004/25)**

## (54) USE OF FLUORINE NMR FOR HIGH THROUGHPUT SCREENING

VERWENDUNG VON FLUOR-NMR ZUM SCREENING MIT HOHEM DURCHSATZ

MISE EN OEUVRE D'UNE IRM AU FLUOR POUR UN DEPISTAGE A HAUT RENDEMENT

(84) Designated Contracting States:
**AT BE BG CH CY CZ DE DK EE ES FI FR GB GR HU IE IT LI LU MC NL PT RO SE SI SK TR**

(30) Priority: **05.06.2002 US 386897 P**
**17.06.2002 US 389252 P**
**26.07.2002 US 398875 P**
**14.03.2003 US 454766 P**
**14.03.2003 US 454765 P**

(43) Date of publication of application:
**09.03.2005 Bulletin 2005/10**

(73) Proprietor: **NERVIANO MEDICAL SCIENCES S.r.l.**
**20014 Nerviano (MI) (IT)**

(72) Inventors:
- **DALVIT, Claudio**
**I-20154 Milano (IT)**
- **STOCKMAN, Brian, J.**
**Kalamazoo, MI 49008 (US)**
- **FLOCCO, Maria**
**20123 Milano (IT)**
- **VERONESI, Marina**
**I-20149 Milano (IT)**

(74) Representative: **Modiano, Micaela Nadia**
**Modiano Josif Pisanty & Staub Ltd**
**Thierschstrasse 11**
**80538 München (DE)**

(56) References cited:
**WO-A-01/23889**

- **CLAUDIO DALVIT ET AL: 'Fluorine-NMR Competition Binding Experiments for High-Throughput Screening of Large Compound Mixtures' COMBINATORIAL CHEMISTRY AND HIGH THROUGHPUT SCREENING vol. 5, no. 8, December 2002, pages 605 - 611, XP008039096**
- **DALVIT ET AL: 'NMR-bases screening with competition water-ligand observed via gradient spectroscopy experiments: detection of high-affinity ligands' J. MED. CHEM. vol. 45, no. 12, June 2002, pages 2610 - 2614, XP002968313**
- **AKOKA ET AL: 'Concentration measurement by proton NMR using the ERETIC method' ANAL. CHEM. vol. 71, no. 13, July 1999, pages 2554 - 2557, XP002271269**
- **JENKINS ET AL: 'Detection of site-specific binding and co-binding of ligands to human serum albumin using 19F NMR' MOLECULAR PHARMACOLOGY vol. 37, no. 1, 1990, pages 111 - 118, XP009096286**
- **SUN ET AL: 'A (19)F-NMR study of the membrane-binding region of D-lactate dehydrogenase of Escherichia coli' PROTEIN SCIENCE vol. 2, 1993, pages 1938 - 1947, XP002978106**
- **JENKINS B.G.: 'Detection of site-specific binding and co-binding of ligands to macromolecules using 19F NMR' LIFE SCI vol. 48, no. 13, 1991, pages 1227 - 1240, XP002978107**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

Background of the Invention

Many drugs currently on the market were developed from leads identified from high throughput screening (HTS). Targets of therapeutic interest used in HTS are often recombinant proteins produced from cloned genes which can be expressed in different ways. A large compound collection is typically screened against these proteins for the identification of inhibitors.

During the last ten years the size of the proprietary compound collection has increased exponentially as a result of systematic application of combinatorial chemistry to different projects. Combinatorial chemistry nowadays generates large compound libraries that complement other compound libraries available from traditional medicinal chemistry and natural sources. The development and application of robotics and automation have made it feasible to test large numbers of compounds in a short period of time. Several new detection systems are used for the identification of potential lead molecules.

Recently, nuclear magnetic resonance (NMR) has emerged as a powerful method for the detection of small molecules that interact with targets of pharmaceutical interest. Although NMR is not a sensitive technique, it has the advantage that it is less subject to artifacts observed with other systems of detection. Recent developments in cryogenic NMR probe technology have reduced the period of time or the amount of protein necessary for the screening. NMR methods have been used for screening a large compound collection against isotopically labeled proteins. Chemical shift changes of cross peaks in a $^{15}N$-$^{1}H$ HSQC spectrum of the target protein are monitored in the presence of a compound mixture. Deconvolution of the mixture then results in the identification of the molecule interacting with the protein (i.e., the compound responsible for the chemical shift changes). When the three dimensional structure of the protein is known and the sequence specific NMR assignments of the protein backbone resonances have been obtained, the method provides important structural information of the ligand binding site and ligand binding mode.

Another method for performing NMR screening is based on the detection of the ligand resonances. Several NMR parameters have been proposed in the literature as a tool for ligand identification. These methodologies permit rapid deconvolution of the screened mixtures and are particularly suited for the identification of medium to low affinity ligands.

However, these techniques suffer from some drawbacks. First, no structural information regarding the binding site is directly available. Second, high affinity ligands and molecules that bind covalently to the receptor escape detection because of the large excess of the test compound over protein typically used in these experiments. That is, compounds interacting tighter to the protein or compounds that have a slow kinetics will not be detected because the residence time of these compounds within the protein is longer than the window of the mixing time (e.g., 1 to 2 seconds) employed in the NMR experiments. Third, compounds with poor solubilities that are potential ligands are difficult to detect since the method requires the observation of the ligand signals.

Thus, what is needed are additional NMR methods that can be used to detect ligands to target molecules, such as proteins, without the drawbacks associated with typical ligand-observed screening experiments.

The article Dalvit C. et al, "NMR-Based Screening with Competition Water-Ligand Observed via Gradient Spectroscopy Experiments: Detection of High-Affinity Ligands", J. Med. Chem. 2002, 45, 2610-2614 describes a method of identifying a ligand to a target molecule, the method comprising: providing a reference compound that interacts with the target molecule; collecting a 1 D nuclear magnetic resonance spectrum of the reference compound in the presence of the target molecule; providing a test sample comprising at least one test compound; collecting a 1 D nuclear magnetic resonance spectrum of the reference compound in the presence of each test sample and the target molecule; comparing the spectrum of the reference compound in the presence of the target molecule to the spectrum of the reference compound in the presence of each test sample and the target molecule to determine a change in one or more of the reference compound resonances; and identifying at least one test compound that interacts with the target molecule, wherein the test compound displaces the reference compound.

The article Jenkins B.G., "Detection of Site-Specific Binding and Co-Binding of Ligands to Macromolecules using 19F NMR", Life Sciences 1991, 48, 1227-1240 describes advantages of using 19F NMR spectroscopy for observing macromolecular-ligand interactions.

Summary of the Invention

The present invention is related to rational drug design. Specifically, the present invention provides a nuclear magnetic resonance (NMR) method of screening for compounds that interact with a target molecule (e.g., typically a protein). The method involves the use of $^{19}F$ NMR, particularly $^{19}F$ NMR competition binding experiments, to detect the binding interaction.

Competition binding experiments involve the displacement of a reference compound in the presence of a

competitive molecule. Preferably, the reference compound binds to the target molecule with a binding affinity in the micromolar range. Preferably, the test compound interacts with the target molecule with a binding affinity stronger than 1 micromolar (e.g., in the nanomolar range), although compounds binding with binding affinities of weaker than (i.e., more than) 1 micromolar can also be evaluated using the methods of the present invention.

The present methodology, particularly when it involves competition binding experiments, can be used for performing efficient high throughput screening (HTS) based on properly set-up competition binding experiments without the drawbacks associated with typical ligand-observed screening experiments. In addition, the methods provide an estimation of the $K_D$ of the identified ligand using a single point measurement. With this approach it is possible to screen thousands of compounds in a short period of time against protein or DNA and RNA fragments, for example.

The present invention could also find useful applications for rapid screening of chemical mixtures (i.e., mixtures of two or more test compounds) such as plant and fungi extracts. Rapid screening techniques typically involve providing a plurality of test samples, each test sample comprising a mixture of two or more test compounds.

Methods of the present invention involve identifying a ligand to a target molecule using the steps set forth in claim 1.

Preferably, methods of the present invention include a step of identifying the reference compound comprising: collecting a WaterLOGSY nuclear magnetic resonance spectrum of a potential reference compound in the absence of the target molecule; collecting a WaterLOGSY nuclear magnetic resonance spectrum of the potential reference compound in the presence of the target molecule; and comparing the WaterLOGSY spectra to identify whether the potential reference compound interacts with the target molecule.

For certain embodiments of the methods of the present invention, the reference compound is provided in combination with an ERETIC signal with defined linewidth, amplitude, and frequency. For these methods, collecting a 1D $^{19}F$ nuclear magnetic resonance spectrum of the $^{19}F$-labelled reference compound in the presence of the target molecule includes collecting a spectrum of the $^{19}F$-labelled reference compound with the ERETIC signal in the presence of the target molecule; and collecting a 1D $^{19}F$ nuclear magnetic resonance spectrum of the $^{19}F$-labelled reference compound in the presence of each test sample and the target molecule includes collecting a spectrum of the $^{19}F$-labelled reference compound with the ERETIC signal in the presence of each test sample and the target molecule.

For certain embodiments of the methods of the present invention, the $^{19}F$-labelled reference compound is provided in combination with an $^{19}F$-labelled non-interacting compound. For these methods, collecting a 1D $^{19}F$ nuclear magnetic resonance spectrum of the $^{19}F$-labelled reference compound in the presence of the target molecule includes collecting a spectrum of the $^{19}F$-labelled reference compound and the $^{19}F$-labelled non-interacting compound in the presence of the target molecule; and collecting a 1D $^{19}F$ nuclear magnetic resonance spectrum of the $^{19}F$-labelled reference compound in the presence of each test sample and the target molecule includes collecting a spectrum of the $^{19}F$-labelled reference compound and the $^{19}F$-labelled non-interacting compound in the presence of each test sample and the target molecule.

Brief Description of the Drawings

Figure 1. Difference in linewidth due to CSA interaction of the $^{19}F$ signal of a small molecule free in solution and when bound to a large macromolecule as a function of the $^{19}F$ Larmor frequency. This simulation was performed using the last term of equation 1 in the assumption of an axally symmetric CSA tensor with a $^{19}F$ CSA of 100 ppm and a correlation time $\tau_c$ of 200 ps for the small molecule when free in solution. Different correlation times for the macromolecule corresponding to different sizes of the macromolecule were considered (values indicated with the curves) The dashed vertical lines indicate some of the commercially available spectrometers. The value corresponding to the $^1H$ Larmor frequency of these spectrometers are indicated with the vertical lines.

Figure 2. NMR screening and deconvolution performed with 50 $\mu$M of the weak affinity ligand Compound A ($K_D$ = 10 $\mu$M) of the p21 activated kinase. The reference molecule contains a $CF_3$ group bound to a six member aromatic ring. The chemical shifts are referenced to TFA. The two spectra were recorded without (left) and with a spin-echo scheme with $\tau$ = 0.1s (right). The spectra were acquired in the presence of 1.5 $\mu$M of the protein for the spy molecule alone (a), in the presence of a 20 $\mu$M seven compound mixture containing the molecules SPECS AB-323/25048456 (supplied by SPECS, Rijswijk, the Netherlands) ethyl 2-quinoxalinecarboxylate, methyl isoquinoline-3-carboxylate, 7-phenyl-4-pteridinol, 2-amino-6-methylquinazolin-4-ol, 5-methylbenzimidazole and Compound B (b), in the presence of the chemical mixture without Compound B (c), in the presence of only Compound B (d). The spectrum of the reference compound in PBS in the absence of the protein is shown in (e). A total of 128 scans with a repetition time of 3.1s were acquired for each experiment.

Figure 3. One dimensional $^{19}F$ spectra recorded in the presence of the weak-affinity ligand Compound A for the p21 activated kinase and the non-interacting trifluoroacetic acid (TFA) molecule. The chemical shifts are referenced

to TFA. A total of 128 scans with a repetition time of 3.1s were acquired for each experiment. The concentration of Compound A and TFA were 50 and 15 μM, respectively. The spectra were recorded in the absence (a) and the presence of 1.5 μM of the protein (b). The spectrum in ( c) corresponds to the difference of the two spectra in (a) and (b). The only signal present in the difference spectrum originates from the spy molecule.

Figure 4. HTS and deconvolution performed with one Dimensional $^{19}F$ spectra recorded in the presence of the weak-affinity ligand Compound A for the p21 activated kinase and the non-interacting trifluoroacetic acid (TFA) molecule. The chemical shifts are referenced to TFA. (a-d) NMR screening and deconvolution performed with 50 μM of Compound A and 15 μM of TFA. (a) Spectrum recorded in the absence of the protein, (b-d) spectra recorded in the presence of 1.5 μM of the protein. (b) Spectrum recorded in the absence of the mixture, (c) spectrum recorded in the presence of a 20 μM six compound mixture containing the molecules SPECS AB-323/25048456, ethyl 2-quinoxalinecarboxylate, methyl isoquinoline-3-carboxylate, 7-phenyl-4-pteridinol, 2-amino-6-methylquinazolin-4-ol, 5-methylbenzimidazole, (d) spectrum recorded in the presence of the same chemical mixture with the addition of 20 μM Compound B. The presence of the competing molecule Compound B results in almost complete displacement of the reference compound from the protein (d) and the spectrum is similar to the spectrum for the two molecules in PBS (a).

Figure 5. Percentage of molecules containing an F atom within the MDDR library. The search was performed from year 1981 to year 2000 in time intervals of five years. The percentage for each interval is indicated above the bars.

Figure 6. $^{19}F$ spin-echo spectra recorded as a function of the HSA concentration. The $CF_3$ resonance of the control molecule **(2)** is at +15.46 ppm and the $CF_3$ resonance of the spy molecule **(1)** is at +14.62 ppm. The spectra were acquired with a total spin-echo period of 320 ms with an interval between the 180° pulses (2τ) of 40 ms. A total of 96 scans with a repetition time of 3.5s and a spectral width of 25 ppm were acquired for each spectrum. The data were multiplied with an exponential function of 1 Hz before Fourier transformation. The concentration of the two molecules was 25 μM whereas the concentration for HSA was from top to bottom, 0, 300, 500, 700 and 900 nM. The signal intensity ratio I(**1**)/I(**2**) is from top to bottom, 0.86, 0.66, 0.38, 0.21 and 0.07.

Figure 7. $^{19}F$ spin-echo spectra recorded as a function of the HSA concentration. The CF resonance of the reference molecule **(3)** is at -64.06 (lower spectra) and the $CF_3$ resonance of the control molecule **(2)** is at +15.46 ppm (upper spectra). The spectra were acquired with a total spin-echo period of 80 ms with an interval between the 180° pulses (2τ) of 40 ms. A total of 96 scans were recorded for the lower spectra and 64 scans for the upper spectra with a repetition time of 3.5s and a spectral width of 25 ppm. The data were multiplied with an exponential function of 1 Hz before Fourier transformation. The concentration of **(3)** and **(2)** was 50 and 25 μM, respectively whereas the concentration for HSA was from left to right, 0, 150, 300, 450, 600 nM. The signal intensity ratio I**(3)**/I**(2)** at the plotted scale intensity is from left to right, 0.94, 0.69,0.53, 0.36 and 0.25.

Figure 8. Plot of the signal intensity ratio (x axis) of the two $^{19}F$ signals of figure 7 as a function of the fraction of bound reference molecule ([EL]/[$L_{TOT}$]) (y axis). The last point on the right corresponds to the value in the absence of the protein. Two ratios ([EL]/[$L_{TOT}$]) were calculated as previously described using the limits of the ITC-derived $K_D$ value of 41 ± 3.3 μM for **(3)**. Values indicated by circles were calculated with a $K_D$ of 44.3 μM, values indicated by squares were calculated with a $K_D$ of 37.7 μM. The curves represent the best fits of the experimental points.

Figure 9. $^{19}F$ NMR screening performed with the control molecule **(2)** (top) and the spy molecule **(3)** (bottom). The spectra were recorded with a total spin-echo period of 160 ms with an interval between the 180° pulses (2τ) of 40 ms. A total of 96 scans were recorded with a repetition time of 3.5s and a spectral width of 25 ppm. The data were multiplied with an exponential function of 1 Hz before Fourier transformation. The concentration of **(3)** and **(2)** was 50 and 25 μM, respectively. The spectra on the left were recorded in the absence of protein while all the other spectra were recorded in the presence of 600 nM HSA. The latter were recorded in the absence of a chemical mixture (2nd from left), in the presence of 50 μM 5-$CH_3$ D,L Trp and Sucrose (3rd from left) and in the presence of 50 μM 5-$CH_3$ D,L Trp, Sucrose and 25 μM of **(4)** (right).

Figure 10. Detection limits of $^{19}F$ NMR screening. Experiments performed with the control molecule **(2)** (top) and the spy molecule **(3)**. The spectra were recorded with a total spin-echo period of 320 ms (top) and 1.2 s (bottom) with an interval between the 180° pulses (2τ) of 40 ms. A total of 64 (top) and 128 (bottom) scans were recorded with a repetition time of 3.5s and a spectral width of 25 ppm. The data were multiplied with an exponential function of 1 Hz before Fourier transformation. The concentration of **(3)** and **(2)** was 50 and 25 μM, respectively. The spectra on the left were recorded in the absence of protein while all the other spectra were recorded in the presence of only 150 nM HSA. The latter were recorded in the absence of a chemical mixture (2nd from left), and in the presence of a mixture containing 25 μM of **(4)** (right).

Figure 11. $^{19}F$ NMR screening performed in the presence of non-deuterated buffers and detergents. (top) Proton spectrum of a 600 nM solution of HSA in 100mM HEPES and 1% Glycerol and in the presence of 50 μM of the spy molecule **(3)** and 25 μM of the control molecule **(2)**. After water suppression the only visible signals are those of the buffer and glycerol. A total of 128 scans was recorded with a repetition time of 2.7s. (Bottom) $^{19}F$ spectra recorded for the same solution in the absence (1st and 3rd spectra from left) and in the presence (2nd and 4th spectra from

left) of a mixture containing 25 μM **(4)**. The spectra were recorded with a total spin-echo period of 160 ms with an interval between the 180° pulses (2τ) of 40 ms. A total of 64 (left) and 128 (right) scans were recorded with a repetition time of 3.5s and a spectral width of 25 ppm. The data were multiplied with an exponential function of 1 Hz before Fourier transformation.

Figure 12. Structures of Compounds 1-4.

Detailed Description of Illustrative Embodiments of the Invention

The present invention is directed to the use of $^{19}F$ NMR, particularly $^{19}F$ NMR competition binding experiments. That is, the present invention is directed to ligand-based screening (preferably, competition screening) using $^{19}F$ experiments. Fluorine-19 detection has many advantages over proton detection in these experiments.

Fluorine is a favorable nucleus for these experiments because of the significant Chemical Shift Anisotropy (CSA) contribution to the $^{19}F$ transverse relaxation of the ligand signal when bound to a protein. That is, the CSA contribution to the $^{19}F$ transverse relaxation makes the fluorine signal especially responsive to the effects of complex formation with the target. A low to moderate affinity ligand containing an $^{19}F$ atom can be used as a reference molecule for the detection and characterization of new ligands. Also, the detection of fluorine significantly reduces or even eliminates the problem of spectral overlap, which occurs in proton ($^1H$) NMR, as the vast majority of compounds to be tested will not contain a fluorine atom. Like proton NMR, $^{19}F$-NMR is highly sensitive and is amenable to rapid data collection, enabling the high-throughput screening of large compound libraries. Fluorine is often used in drug-design efforts to enhance the pharmacokinetic properties of biologically active compounds. As about 12% of the molecules comprising the Available Chemical Directory Screening Compounds (ACD-SC) contain a fluorine atom, a reference compound for the competition screening can typically be obtained without recourse to chemical synthesis. In fact, the fluoro-benzene and the trifluoromethyl-benzene moiety are found in approximately 150,000 and approximately 40,000 molecules, respectively.

Competition binding experiments involve the displacement of a reference compound in the presence of a competitive molecule. Preferably, the reference compound binds to the target molecule with a binding affinity in the micromolar range. Preferably, the test compound binds to the target molecule with a binding affinity stronger than (i.e., less than) 1 micromolar (e.g., in the nanomolar range), although compounds binding with a binding affinity weaker than (i.e., more than) 1 micromolar can also be evaluated using the methods of the present invention.

Although the methods described herein are particularly useful for identifying ligands that are relatively strong binders to the target molecule, they can be used for identifying ligands of a wide range of binding affinities. The relatively strong binders are typically defined as those having a dissociation binding constant $K_D$ of less than about 1 micromolar, preferably less than about 500 nM, more preferably less than about 100 nM.

Competition binding experiments are not limited to screening libraries of compounds that are highly soluble in aqueous buffer. Typically, only the reference compound needs to be water soluble, and compounds with limited solubility can still be detected by their indirect effect on the signal of the reference compound. The reference molecule (that, for its role, is called the *spy molecule*) is generally water-soluble in order to avoid artefacts originating from possible non-specific interactions of the reference molecule with the receptor and with molecules of the mixtures to be screened. Titration NMR experiments with the reference molecule are typically first performed either at different ligand concentrations and fixed protein concentration or different protein concentrations and fixed ligand concentration (C. Dalvit et al., J. Am. Chem. Soc.,124, 7702-7709 (2002)). These experiments are used for the optimization of the screening setup conditions and for deriving the binding constant of the identified NMR hits from a single point measurement. Strong ligands are easily identified due to their large effect on the spy molecule. However, one limitation of the current $^1H$ NMR competition screening methods may be represented by the occurrence of spectral overlap between the reference and test compounds particularly when large chemical mixtures are screened. The use of NMR detection of other nuclei (e.g., $^{19}F$) reduces significantly these problems.

According to the invention, the following steps are used: providing an $^{19}F$-labelled reference compound that interacts with the target molecule; collecting a 1D $^{19}F$ nuclear magnetic resonance spectrum of the $^{19}F$-labelled reference compound in the presence of the target molecule; providing at least one test sample (preferably a plurality of test samples), each test sample comprising at least one test compound; collecting a 1D $^{19}F$ nuclear magnetic resonance spectrum of the $^{19}F$-labelled reference compound in the presence of each test sample and the target molecule; comparing the spectrum of the $^{19}F$-labelled reference compound in the presence of the target molecule to the spectrum of the $^{19}F$-labelled reference compound in the presence of each test sample and the target molecule to determine a change in one or more of the $^{19}F$-labelled reference compound resonances; and identifying at least one test compound that interacts with the target molecule, wherein the test compound displaces the $^{19}F$-labelled reference compound (typically, this results because the test compound has a binding affinity at least as tight as that of the reference compound).

Typically, a change in one or more of the $^{19}F$-labelled reference compound resonances involves an increase in signal intensity in at least one reference resonance. Preferably, a change in one or more of the $^{19}F$-labelled reference

compound resonances involves a sharpening of at least one reference resonance.

If desired, before the acquisition of the $^{19}F$ spectra, spin-echo type filters can be applied, as described in the Examples Section.

The optimum experimental conditions for any of the methods described herein can be determined as described in the Examples Section. Specifically, this typically involves the following steps being carried out prior to collecting a 1D $^{19}F$ nuclear magnetic resonance spectrum of the $^{19}F$-labelled reference compound in the presence of the target molecule for use in the comparing step: collecting 1D $^{19}F$ nuclear magnetic resonance spectra of the $^{19}F$-labelled reference compound in the presence of the target molecule at different concentrations of the target molecule or at different concentrations of the $^{19}F$-labelled reference compound. The information collected is used to determine the optimum experimental conditions for identifying at least one test compound that interacts with the target molecule.

A wide variety of pulse sequences can be used when collecting the 1D $^{19}F$ NMR spectrum of the $^{19}F$-labelled reference compound in the presence of each test sample and the target molecule. For effective comparison of spectra, it is desirable to have the same experimental conditions; however, target compound and $^{19}F$-labelled reference molecule concentrations can be varied as long as the graphs with the titration experiments have been generated before the screening. Generally, the temperature and buffer conditions are the same, because a change in these experimental conditions can affect the binding constant of the reference compound.

In the generalized method described above for mixtures of two or more test compounds, identifying at least one test compound may preferably involve recording separate 1D $^{19}F$ nuclear magnetic resonance spectra of the $^{19}F$-labelled reference compound in the presence of each test compound and the target molecule. This is followed by comparing the spectrum of the $^{19}F$-labelled reference compound in the presence of the target molecule to the spectrum of the $^{19}F$-labelled reference compound in the presence of each test compound and the target molecule to determine a change in the selected $^{19}F$-labelled reference compound resonance. The pulse sequences of these experiments are generally the same. Such experiments are typically referred to by those of skill in the art as deconvolution experiments.

The dissociation constant (i.e., binding affinity) of a test compound and/or a reference compound can be determined using NMR techniques if desired, although other well-known techniques can be used as well (e.g., isothermal titration calorimetry). Preferably, the reference compound binding affinity is evaluated using isothermal titration calorimetry or fluorescence spectroscopy, the specific details of which are well-known to one of skill in the art and are described in the Examples Section.

For example, in one NMR-based method, in addition to the above-listed steps in the generalized method, 1D $^{19}F$ nuclear magnetic resonance spectra of the $^{19}F$-labelled reference compound in the presence of the target molecule at different concentrations of the $^{19}F$-labelled reference compound can be collected. Alternatively or additionally, 1D $^{19}F$ nuclear magnetic resonance spectra of the $^{19}F$-labelled reference compound in the presence of the target molecule at different concentrations of the target molecule can be collected. This information can be used to determine the dissociation constant of the test compound as described in the examples.

For increasing the precision of any one method of the present invention, various techniques can be used. Typically, an internal control can be used, which can be a non-interacting compound.

An alternative to the use of a non-interacting molecule is the use of the ERETIC method (S. Akoka et al., Anal. Chem., 71, 2554-2557 (1999); and V. Silvestre et al., S. Anal, Chem., 73, 1862-1868 (2001). This technique relies on the electronic generation of a signal of a defined frequency, linewidth and amplitude. A pseudo-FID is acquired with the FID originating from the sample. The amplitude of this artificial signal is adjusted to become comparable to the intensity of the signal of the reference compound recorded in the absence of the protein. This amplitude value is then used for the titration and NMR-screening experiments and the signal intensity ratio of the real and artificial signal is measured. Adding an ERETIC signal is like adding a fake signal to normalize the signals.

For certain embodiments of the methods of the present invention, the reference compound is provided in combination with an ERETIC signal with defined linewidth, amplitude, and frequency. For these methods, collecting a 1D $^{19}F$ nuclear magnetic resonance spectrum of the $^{19}F$-labelled reference compound in the presence of the target molecule includes collecting a spectrum of the $^{19}F$-labelled reference compound with the ERETIC signal in the presence of the target molecule; and collecting a 1D $^{19}F$ nuclear magnetic resonance spectrum of the $^{19}F$-labelled reference compound in the presence of each test sample and the target molecule includes collecting a spectrum of the $^{19}F$-labelled reference compound with the ERETIC signal in the presence of each test sample and the target molecule.

For certain embodiments of the methods of the present invention, the $^{19}F$-labelled reference compound is provided in combination with an $^{19}F$-labelled non-interacting compound. For these methods, collecting a 1D $^{19}F$ nuclear magnetic resonance spectrum of the $^{19}F$-latielled reference compound in the presence of the target molecule includes collecting a spectrum of the $^{19}F$-labelled reference compound and the $^{19}F$-labelled non-interacting compound in the presence of the target molecule; and collecting a 1D $^{19}F$ nuclear magnetic resonance spectrum of the $^{19}F$-labelled reference compound in the presence of each test sample and the target molecule includes collecting a spectrum of the $^{19}F$-labelled reference compound and the $^{19}F$-labelled non-interacting compound in the presence of each test sample and the target molecule. Such non-interacting compounds act as controls in that they do not bind to the target molecule

at the concentrations evaluated.

In combination with the competition binding experiments of the present invention, the WaterLOGSY method can be used to identify the reference compound, as well as other methods such as spectroscopic or biochemical assays, which are well known to one of skill in the art. Preferably, the reference compound can be identified by the following steps: collecting a WaterLOGSY nuclear magnetic resonance spectrum of a potential reference compound in the absence of the target molecule; collecting a WaterLOGSY nuclear magnetic resonance spectrum of the potential reference compound in the presence of the target molecule; and comparing the WaterLOGSY spectra to identify whether the potential reference compound interacts with the target molecule.

The WaterLOGSY method (also referred to as the Water-Ligand Observed via Gradient Spectroscopy Y) is based on the transfer of magnetization from the protons of bulk water to the protons of compounds that interact with target molecules (e.g., proteins). Using WaterLOGSY techniques, binding compounds are distinguished from nonbinders by the opposite sign of their water-ligand nuclear Overhauser effects (NOEs). The WaterLOGSY method is described in greater detail in International Publication No. WO 01/23330 (published April 5, 2001), in C. Dalvit et al., J. Biomol. NMR, 18, 65-68 (2000).

The target molecules that can be used in the methods of the present invention include a wide variety of molecules, particularly macromolecules, such as polypeptides (preferably, proteins), polynucleotides, organic polymers, and the like. These can be within a living cell or in a lysate.

"Polynucleotide" as used herein refers to a polymeric form of nucleotides of any length, either ribonucleotides or deoxynucleotides, and includes both double- and single-stranded DNA and RNA. A polynucleotide may include both coding and non-coding regions, and can be obtained directly from a natural source (e.g., a microbe), or can be prepared with the aid of recombinant, enzymatic, or chemical techniques. A polynucleotide can be linear or circular in topology. A polynucleotide can be, for example, a portion of a vector, such as an expression or cloning vector, or a fragment.

"Polypeptide" as used herein refers to a polymer of amino acids and does not refer to a specific length of a polymer of amino acids. Thus, for example, the terms peptide, oligopeptide, protein, and enzyme are included within the definition of polypeptide. This term also includes post-expression modifications of the polypeptide, for example, glycosylations, acetylations, phosphorylations, and the like.

The reference compound is one that interacts with the selected target molecule with a binding affinity sufficiently low. Relatively weakly interacting reference compounds are typically defined as those having a dissociation binding constant $K_D$ of at least 10 micromolar.

The test compounds that can be evaluated can be any of a wide variety of compounds, which potentially have a wide variety of binding affinities to the target. Significantly, the method of the present invention has the ability to detect compounds that are relatively strong binders. The relatively strong binders are typically defines as those having a dissociation binding constant $K_D$ of less than about 1 micromolar. Compounds that can be screened using the method of the present invention include, for example, plant extracts, fungi extracts, other natural products, and libraries of small organic molecules.

The present invention can screen for ligands from a library of compounds that have a broad range of solubilities (the methods are particularly amendable to compounds having very low solubilities). Significantly and advantageously, for certain embodiments, the present invention preferably involves carrying out a binding assay at relatively low concentrations of target (i.e., target molecule). Thus, preferred embodiments of the present invention allow for the detection of compounds that are only marginally soluble. Typically these compounds have a solubility in water of no greater than about 10 $\mu$M.

Preferably, the concentration of each test compound in each sample is no greater than about 100 $\mu$M, although higher concentrations can be used if desired. However, a significant advantage of the method of the present invention is that very low ligand concentrations (e.g., no greater than about 10 $\mu$M) can be used.

The exact concentrations and ratios of test compound to target molecule used can vary depending on the size of the target molecule, the amount of target molecule available, the desired binding affinity detection limit, and the desired speed of data collection. Preferably, the concentration of target molecule is about 100 nM to about 10 $\mu$M.

The solvents used for the test mixtures can be any of a wide variety as long as they do not degrade (e.g., denature) the target. Typically water and DMSO are used. Protonated solvents and detergents can be used.

If desired other components (e.g., buffers) can be added to the test mixtures for certain advantage, as is well known to one of skill in the art.

The present invention could also find useful applications for rapid screening of chemical mixtures (i.e., mixtures of two or more test compounds). Rapid screening techniques typically involve providing a plurality of test samples, each test sample comprising a mixture of two or more test compounds.

Once a ligand (preferably a high affinity ligand) has been identified and confirmed, its structure is used to identify available compounds with similar structures to be assayed for activity or affinity, or to direct the synthesis of structurally related compounds to be assayed for activity or affinity. These compounds are then either obtained from inventory or synthesized. Most often, they are then assayed for activity using enzyme assays. In the case of molecular

targets that are not enzymes or that do not have an enzyme assay available, these compounds can be assayed for affinity using NMR techniques similar to those described above, or by other physical methods such as isothermal denaturation calorimetry. Compounds identified in this step with affinities for the molecular target of about 1.0 x $10^{-6}$ M or better are typically considered lead chemical templates.

In some instances, ligand binding is further studied using more complex NMR experiments or other physical methods such as calorimetry or X-ray crystallography.

Cryoprobe technology optimized for $^1H$ and $^{19}F$ detection could further enhance the throughput of this screening process. In this case, the limiting factor will be the time required to change the sample, equilibrate the sample temperature, and shim the sample.

Examples

Objects and advantages of this invention are further illustrated by the following examples, but the particular materials and amounts thereof recited in these examples, as well as other conditions and details, should not be construed to unduly limit this invention.

MATERIALS AND METHODS

For the first set of experiments in Example (I), the kinase domain (MW approximately 34000) of a Serine/Threonine p21-activated kinase was expressed as a GST fusion protein in E. Coli and purified to homogeneity after removal of the GST tag. NMR samples were in phosphate-buffered saline (PBS, code: P-3813, Lot 100K8211 from Sigma) pH 7.4. $D_2O$ was added to the solution (8% final concentration) for the lock signal. The small molecules were prepared in concentrated stock solutions in deuterated DMSO and stored at 253 K.

For the second set of experiments in Example (II), fatty acid free human serum albumin (A-3782) was purchased from Sigma and used without further purification. NMR samples were in phosphate-buffered saline (PBS, code: P-3813, Lot 100K8211 from Sigma) pH 7.4 in the presence of 5 $\mu$M EDTA. $D_2O$ was added to the solution (8% final concentration) for the lock signal. The small molecules were prepared in concentrated stock solutions in either deuterated DMSO or water and stored at 253 K.

*NMR*

For the first set of experiments in Example (I), all NMR spectra were recorded at 293 K with a Varian Inova 600 MHz (564 MHz for $^{19}F$) NMR spectrometer equipped with a Sample Management System (SMS) autosampler. Water suppression in the $^1H$ detected experiments was achieved with the excitation sculpting sequence (T.-L. Hwang, J. Magn. Reson. A, 112, 275-279 (1995)). The two water selective 180° square pulses and the four pulsed field gradients of the scheme were 2.6 and 1 millisecond (ms) in duration, respectively.

For the second set of experiments in Example (II), all NMR spectra were recorded at 300 K with a Bruker Avance 600 NMR spectrometer operating at a $^{19}F$ Larmor frequency of 564 MHz. A dual coil $\{^{19}F\}$-$\{^1H\}$ probe was used with the inner coil tuned to $^{19}F$ and the outer coil tuned to $^1H$ frequency. The fluorine background of these probes does not interfere with the measurements. These signals are broad and therefore are not visible in the spectra of the reference and control molecule recorded with a spin-echo scheme. All the spectra were recorded with a weak Waltz-16 proton decoupling applied during the acquisition period. Typically 4-8 dummy scans were recorded for temperature equilibration. Carr-Purcell-Meibom-Gill schemes of different length and long $2\tau$ interval were used before the acquisition period. Chemical shifts were referenced to trifluoroacetic acid.

*Fluorescence*

Fluorescence measurements were acquired on a Jasco J-715 spectropolarimeter using an auxillary photomultiplier tube positioned perpendicular to the excitation beam. The excitation wavelength was 310 nm (with a 5 nm bandwidth) and a 385 nm cut-off filter was employed. Affinity measurements were made using the same source of fatty acid free HSA as used for NMR experiments. Analyte and HSA solutions were prepared in phosphate-buffered saline (PBS, code: P-3813, Lot 100K8211 from Sigma) pH 7.4 in the presence of 5 $\mu$M EDTA. The buffer was filtered through a 0.2 $\mu$m filter prior to use. Albumin affinity was determined by aliquoting 2.0 mL of a 3 $\mu$M solution of analyte into a quartz cuvette, pathlength of 1.0 cm, and titrating the solution with HSA (stock concentration of 250 $\mu$M).

*ITC Experiments*

Isothermal titration calorimetry experiments were performed using an OMEGA titrating microcalorimeter from

Microcal, Inc. (Northampton, MA). The titrating microcalorimeter consisted of a sample and reference cell held in an adiabatic enclosure. The reference cell was filled with PBS. A 23 μM solution of HSA in PBS +2% DMSO was placed in the 1.37 mL sample cell. Analyte at 0.8 mM in the same buffer was held in a 250 μL syringe. Thirty injections (8 μL each and 12 seconds/injection) of analyte were made by a computer controlled stepper motor into the sample cell held at 25°C. The syringe stir rate was 400 rpm. Heat adsorbed or released with each injection was measured by a thermoelectric device connected to a Microcal nanovolt preamplifier. Titration isotherms for the binding interactions were comprised of the differential heat flow for each injection. Heat of dilution obtained by injecting analyte into PBS was negligible. Binding isotherms were fit to a single binding site model (T. Wiseman et al., Anal. Biochem., 179, 131-137 (1989)) using an iterative nonlinear least-squares algorithm included with the instrument.

Example I.

RESULTS AND DISCUSSION

*$^{19}F$ Relaxation Theory*

The longitudinal relaxation of $^{19}F$ is not a good parameter for the competition binding experiments since it lacks the direct $\tau_c$ dependence necessary for identifying small molecules interacting with a macromolecule. However, the transverse relaxation rate $R_2$ represents an excellent parameter since it contains spectral densities calculated at 0 frequency (M. Goldman, Quantum Description of High-Resolution NMR in Liquids, Clarendon Press, Oxford, (1988)) for the heteronuclear $^{19}F$ - $^{1}H$ dipolar interactions and for the $^{19}F$ chemical shift anisotropy (CSA) interaction as described by the equation:

$$R_2^F = \frac{\gamma_F^2\gamma_H^2\hbar^2\tau_c}{20}\sum_{H_i}\frac{1}{r_{FHi}^6}\left\{4+\frac{1}{1+(\omega_F-\omega_H)^2\tau_c^2}+\frac{3}{1+\omega_F^2\tau_c^2}+\frac{6}{1+\omega_H^2\tau_c^2}+\frac{6}{1+(\omega_F+\omega_H)^2\tau_c^2}\right\}+$$
$$\frac{2}{15}\Delta\sigma^2 B_0^2\gamma_F^2\tau_c\left\{\frac{2}{3}+\frac{1}{2(1+\omega_F^2\tau_c^2)}\right\}$$

(1)

The $H_i$ correspond to all the protons of the reference compound and of the protein close in space to the fluorine atom, $\Delta\sigma$ is the CSA of the $^{19}F$ atom and $B_0$ is the strength of the magnetic field, $\gamma_H$ and $\gamma_F$ are the proton and fluorine gyromagnetic ratios, respectively, $\omega_H$ and $\omega_F$ are the proton and fluorine Larmor frequencies, respectively, $\tau_c$ is the correlation time and $r_{FHi}$ is the internuclear distance between proton $H_i$ and the fluorine atom.

Owing to the large CSA of $^{19}F$ (as much as few hundreds ppm) (J.T. Gerig, Methods in Enzymol., 177, 3-23 (1989); and J.T. Gerig, Prog. NMR Spectrosc., 26, 293-370 (1994)) it will contribute significantly to the transverse relaxation of the fraction of bound ligand. CSA contribution to relaxation is directly proportional to the square of the magnetic field. Therefore, the effect is more pronounced at higher magnetic fields. This can be appreciated in the simulation of Figure 1 where the difference in $^{19}F$ linewidth due to CSA contribution for a small molecule free in solution and when bound to a large macromolecule of different sizes is plotted as a function of the Larmor frequency. A $\Delta\sigma$ of 100 ppm and a $\tau_c$ of 200 ps for the small molecule free in solution were used for the simulation. As can be appreciated from Figure 1 the strongest magnetic fields available today are not optimal for the $^{19}F$ experiments of strongly protein-bound ligands and of proteins selectively labeled with $^{19}F$ due to the strong contribution of CSA. In contrast, the strong magnetic fields are particularly suited for the competition binding HTS experiments performed with a weak affinity reference molecule. The difference in $^{19}F$ resonance linewidth of the reference molecule between the free and bound state increases with the strength of the magnetic field. In addition, the chemical shift difference for the fluorine ligand resonance between the free and bound state ($\delta_{free}$ - $\delta_{bound}$) becomes larger. This results in a significant contribution of the exchange term $R_{2,\ ex}$

$$R_{2,ex} = \frac{[EL]}{[L_{TOT}]}\left(1 - \frac{[EL]}{[L_{TOT}]}\right)^2 \frac{4\pi^2(\delta_{free} - \delta_{bound})^2}{K_{-1}} \qquad (2)$$

to the $^{19}F$ linewidth of the reference compound (J.W. Peng,. J. Magn. Reson., 153, 32-47 (2001)). $[EL]/[L_{TOT}]$ is the fraction of bound ligand and $1/K_{-1}$ is the residence time of the ligand bound to the protein.

*Selection of the Reference Compound and Screening Parameters*

As the NMR screening experiments are typically carried out with a 10-100 fold excess of ligand over protein (resulting in only a small fraction of bound ligand), the terms in the preceeding section ($\Delta\sigma$, Bo, $\delta_{free}$ - $\delta_{bound}$, $1/K_{-1}$, etc.) become important in the selection of the reference compound. The chemical shift of the $^{19}F$ signal of the reference compound bound to the protein can be very different when compared to the chemical shift of the free ligand due to the contribution of the protein induced shift (J.T. Gerig, Prog. NMR Spectrosc., 26, 293-370 (1994); and J. Feeney et al., J. Am. Chem. Soc., 118, 8700-8706 (1996)). Therefore even molecules having a medium binding affinity in the $\mu$M range could display a $^{19}F$ spectrum with two distinct resonances, one for the bound form and the other for the free form. This arises from the fact that the difference in chemical shift of the two resonances is larger when compared to the exchange rate between the free and bound species. In the HTS only the signal at the frequency of the free ligand will be monitored since the concentration of the protein is to low (few hundreds nM to 1-5 $\mu$M) for allowing the observation of the very broad resonance of the bound form.

In order to assess the suitability of any given fluorinated ligand for competition screening and to determine the optimal experimental parameters, titration experiments for the candidate molecule are performed as a function of the fraction of bound ligand (C. Dalvit et al., J. Am. Chem. Soc., 124, 7702-7709 (2002)) by acquiring 1D $^{19}F$ $R_2$ filtered experiments or simply 1D $^{19}F$ experiments. For better sensitivity the experiments are recorded with $^{1}H$ decoupling during the acquisition period. Candidate compounds and experimental conditions are examined for their sensitivity and for the presence of a single fluorine resonance that exhibits significant perturbations upon binding to the target.

Once a suitable ligand has been identified and experimental conditions established, screening can then be carried out by monitoring changes in the transverse relaxation (either via the $R_2$ filtered experiments performed with CPMG or spin-echo schemes or simply by analysis of the linewidth) of the $^{19}F$ signal of the reference molecule as shown in Figure 2.

In this case, 50 $\mu$M of the moderate affinity ligand Compound A ($K_D$ = 10 $\mu$M) was used as the reference compound in the presence of 1.5 $\mu$M of PAK4. A significant increase in the signal of Compound A after the spin-echo scheme was observed in the presence of a compound mixture containing a high-affinity ligand (Compare Figure 2a with 2b). This increase in signal indicates a decrease in binding of the reference molecule Compound A due to competition wiht a molecule contained in the mixture. Simple deconvolution experiments (2c and 2d) allows for the identification of Compound B as the active molecule.

When spin-echo type sequences are used, it is recommended to use a sufficiently long delay between the hard 180° pulses in order to take advantage of the exchange term of equation (2). This is possible since there are no homonuclear scalar couplings and the evolution under the heteronuclear scalar couplings is refocused. However, the delay should not be very long in order to avoid relaxation deriving from the inhomogeneity of the static magnetic field.

In addition to the fluorinated reference compound, it is advantageous to include a fluorinated small molecule that does not interact with the receptor (a control compound). Figure 3 shows this principle where the $^{19}F$ spectra of the spy molecule and of the non-interacting molecule are recorded in the absence and presence of the protein. While the signal of the spy molecule undergoes spectral changes, the signal of the small molecule will not change. This can be appreciated in the difference spectrum of Figure 3. The signal of the non-interacting molecule represents an internal reference that can be used for calibrating with a single experiment the changes in the signal of the spy molecule. It should be pointed out that even if the small molecule had a weak interaction (mM range) with the receptor this would not interfere with the measurements. The concentration of the small molecule (10-30 $\mu$M) is orders of magnitude smaller when compared to the weak binding constant and therefore the fraction of compound bound to the receptor is negligible. In order to prove this trifluoroacetic acid (TFA) was chosen as the small molecule non-interacting with the receptor. It should be noted that some compounds in the screening may contain traces of TFA. Thus, while valid for the proof of concept presented here, an alternative control compound should be selected for more general application. The utility of using both a spy molecule and a control compound for lead identification through HTS and deconvolution is shown in Figure 4. The six compound mixture does not affect the linewidth of the spy molecule resonance (Figure 4c) and the signal of the reference compound Compound A is clearly less intense when compared to the signal of TFA. However,

the presence of a strong competing molecule (Compound B) in the seven compound mixture results in a sharpening of the resonance of the spy molecule (Figure 4d) and the two signals have now comparable intensity.

As described in C. Dalvit et al., J. Am. Chem. Soc., 124, 7702-7709 (2002), it is possible to derive the binding constant of the identified NMR-hit from the signal intensity ratio of the two $^{19}F$ resonances plotted as a function of the fraction of bound ligand and the measurement of the signal intensity change of the reference molecule in the presence of a competing molecule. In this specific case the binding constant for the NMR-hit Compound B was determined to be 200 +/- 100 nM. When $^{19}F$ experiments are used for the HTS it is important also to record the $^{1}H$ spectra in order to estimate the concentration of the compounds comprising the chemical mixtures and therefore derive a reliable value for the binding constant of the NMR hits.

A $^{1}H$ to $^{19}F$ NOE step can also be applied in the $^{19}F$ experiments before the acquisition period in order to transfer magnetization from the protons to the fluorine spin. This step can be performed in different ways. An enhancement of the $^{19}F$ signal is observed for a small molecule not interacting with the large receptor: A very weak signal enhancement or a signal reduction, depending on the fraction of bound ligand, protein correlation time and on how the NOE step is performed is observed for a molecule interacting weakly with the receptor. These differences of the heteronuclear NOE can be used constructively in competition binding HTS experiments. When the *spy molecule* is displaced from the receptor in the presence of a competing molecule its $^{19}F$ signal becomes more intense because of a smaller linewidth and signal enhancement via heteronuclear NOE.

Example II.

RESULTS AND DISCUSSION

*Theory*

The sensitivity of $^{19}F$ NMR signal is proportional to $(\gamma_F/\gamma_H)^3$ where $\gamma_F$ and $\gamma_H$ are the gyromagnetic ratio of fluorine and proton, respectively. Owing to the fact that $^{19}F$ is the only stable fluorine isotope and has spin ½ its sensitivity is high, i.e. 0.83 times that of the proton. Fluorine signals appear as singlet resonances in the presence of proton decoupling and are therefore intense.

The $^{19}F$ transverse relaxation represents an excellent parameter to be monitored for screening performed with competition binding experiments. A dipolar interaction between fluorine and a proton located at a certain distance is very similar in magnitude (0.88 times) to a dipolar interaction between two protons separated by the same distance. Therefore the dipolar contributions to the linewidth of a fluorine or proton signal of a reference molecule are similar. The transverse relaxation rate $R_2$ of the fluorine signal has an additional contribution originating from the large CSA interaction of the $^{19}F$ atom and is given by the following equation (D. Canet, Nuclear Magnetic Resonance Concepts and Methods, John Wiley & Sons, Chichester, (1996)):

$$R_2^{CSA} = \frac{2}{15}\Delta\sigma^2\left(1+\frac{\eta_{CSA}^2}{3}\right)B_0^2\gamma_F^2\tau_c\left\{\frac{2}{3}+\frac{1}{2(1+\omega_F^2\tau_c^2)}\right\} \quad (3)$$

where $\Delta\sigma$ is the CSA of the $^{19}F$ atom and is given by $\Delta\sigma = \sigma_{zz} - (\sigma_{xx} + \sigma_{yy})/2$ . The different σ's are the components of the chemical shift tensor. The asymmetry parameter $\eta_{CSA} = (3/2)(\sigma_{xx} - \sigma_{yy})/\Delta\sigma$ and for an axially symmetric chemical shift tensor $\eta_{CSA} = 0$. $B_0$ is the strength of the magnetic field, $\gamma_F$ is the fluorine gyromagnetic ratio, $\omega_F$ is the fluorine Larmor frequency, and $\tau_c$ is the correlation time.

As discussed above in the first set of experiments (I), simulation performed assuming an axially symmetric CSA tensor and assuming an equal CSA for the free and bound state of a ligand, as shown in Figure 1, indicates that the difference in linewidth of the $^{19}F$ signal of the reference molecule between the free and bound state from just the CSA contribution alone can be very large (C. Dalvit et al., Comb. Chem. HTS, 5, 605-611 (2002) and Example (I) above). This difference increases with the size of the receptor and with the square of the magnetic field strength. High magnetic fields can lead to extremely broad linewidths (>200 Hz) for fluorine signals of either macromolecules (e.g., a protein selectively labeled with $^{19}F$) or strongly protein-bound ligands (W.E. Hull et al., J. Mol. Biol., 98, 121-153 (1975); J.T. Gerig, Methods in Enzymol., 177, 3-23 (1989); and J.T. Gerig, Prog. NMR Spectrosc., 26, 293-370 (1994)). Such linewidths make the direct detection of fluorine resonances of the macromolecule or high-affinity ligands impractical for the purposes of screening. In contrast, the strong magnetic fields are particularly suited for competition binding experiments performed with a weak affinity reference molecule where the population averaging between the free and bound states results in an observed linewidth that can be manipulated and monitored (C. Dalvit et al., Comb. Chem. HTS, 5, 605-611 (2002) and Example (I) above).

The pulse sequences typically used employ a Carr-Purcell Meibom Gill (CPMG) spin-echo scheme (H.Y. Carr et al., Phys. Rev., 94, 630-638 (1954); and S. Meiboom et al., Rev. Sci. Instrum., 29, 688 (1958)) before the acquisition period. The signal intensity of the reference molecule at the end of the spin-echo scheme $I_{(n2\tau)}$ is given by the following equation (T.C. Farrar et al., Pulse and Fourier Transform NMR, Introduction to Theory and Methods, Academic Press, New York, 1971):

$$I_{(n2\tau)} = I_0 e^{-\gamma_F^2 G^2 D_{obs} (n2\tau) \frac{\tau^2}{3}} e^{-n2\tau R_{2,obs}} \qquad (4)$$

where $I_0$ is the signal intensity after the initial 90° pulse, $2\tau$ is the interval between the train of 180° pulses, G is the inhomogeneity of the static magnetic field, $\gamma_F$ is the gyromagnetic ratio of fluorine, and n is the number of cycles of the spin-echo scheme. $D_{obs}$, the observed translation diffusion coefficient for the weak affinity reference molecule, is given by the equation:

$$D_{obs} = \frac{[EL]}{[L_{TOT}]} D_{bound} + \left(1 - \frac{[EL]}{[L_{TOT}]}\right) D_{free} \qquad (5)$$

where $D_{bound}$ and $D_{free}$ are the diffusion coefficients of the reference molecule in the bound and free states, respectively. $[EL]/[L_{TOT}]$ and $(1-[EL]/[L_{TOT}])$ are the fraction of bound and free ligand, respectively.

$R_{2,obs}$, the transverse relaxation rate for the weak affinity reference molecule, is given by the equation:

$$R_{2,obs} = \frac{[EL]}{[L_{TOT}]} R_{2,bound} + \left(1 - \frac{[EL]}{[L_{TOT}]}\right) R_{2,free} + \frac{[EL]}{[L_{TOT}]} \left(1 - \frac{[EL]}{[L_{TOT}]}\right)^2 \frac{4\pi^2 \left(\delta_{free} - \delta_{bound}\right)^2}{K_{-1}} \qquad (6)$$

where $R_{2,bound}$ and $R_{2,free}$ are the transverse relaxation rate constants for the ligand in the bound and free states, respectively. The last term is the exchange term where $\delta_{bound}$ and $\delta_{free}$ are the isotropic chemical shifts of the fluorine resonance of the reference molecule in the bound and free states, respectively and $1/K_{-1}$ is the residence time of the ligand bound to the protein. Equation (6) is valid only when the experiments are performed with a long $2\tau$ period (where $\tau >> 1/K_{-1}$). Experiments recorded with $\tau < 5/K_{-1}$ result in a reduced contribution of the exchange term to the observed transverse relaxation rate (Z. Luz et al., J. Chem. Phys., 39, 366-370 (1963); and A. Allerhand et al., H.S. J. Chem. Phys., 41, 2115-2126 (1964)).

Therefore screening is performed by using a long $2\tau$ period. This is possible because the evolution under the heteronuclear $^1H$-$^{19}F$ scalar couplings is refocused at the end of the scheme. However, the $2\tau$ period should not be very long in order to reduce signal attenuation originating from the spatial diffusion of the reference molecule (i.e., first exponential term of equation (4)).

*Selection of the spy and control molecules*

Table 1 reports the frequency of molecules containing a fluorine atom in three different commercially available chemical libraries. The table contains also the number of two substructures, monofluoro-benzene and trifluoromethyl-benzene, often used in these experiments. The large number of molecules containing a fluorine atom makes the selection of the spy and control molecules an easy task without recourse to chemical synthesis.

| Table 1: Frequency of F containing molecules in different commercially available libraries. ACD-SC (Available Chemical Directory of Screening Compounds), MDDR (MDL Drug Data Report), NCI (National Cancer Institute) | | | |
|---|---|---|---|
| **Compound Collection** | **molecules with F** | 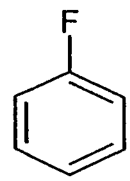 | 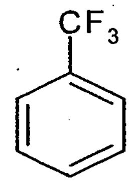 |
| **ACD-SC** | **~12%** | **153000** | **43000** |
| **MDDR** | **~15%** | **11000** | **3000** |
| **NCI** | **~4%** | **3000** | **1000** |

An interesting feature emerging from Table 1 is the large number of fluorine containing molecules present in the MDDR library. A chronological search within this library, as shown in Figure 5, demonstrates that over the last 20 years the percentage of compounds in development containing at least one fluorine atom has doubled. A steady increase from 10.9% in the 1981-1985 period to 19.4% in the 1996-2000 period is observed. The fluorine atom has been increasingly introduced in the process of lead optimization for improving potency, physical-chemical properties and metabolic stability against enzyme attack.

In the selection of the two molecules particular care should be paid to their solubility. The presence of a fluorine atom increases the lipophilicity of a compound. Molecules that are not very soluble in aqueous solution are not suitable for screening experiments since they might bind in a non-specific manner to the receptor. Therefore proton and fluorine spectra and proton WaterLOGSY spectra for the potential spy and control molecules are recorded in the absence of protein at a concentration typically 2 to 4 times higher (i.e., 100 to 200 μM) than the concentration used in the screening process. Only molecules that according to the NMR spectra are soluble and do not aggregate at these concentrations are considered as potential candidates for the reference and control molecules used for the screening.

*Molecules with a $CF_3$ group*

Reference molecules containing a $CF_3$ group have the advantage of high sensitivity of the fluorine signal. Typical spin-echo $^{19}F$ spectra of the reference molecule 5-[1-methyl-3(trifluoromethyl)-1H-pyrazol-5-yl]-2-thiophenecarboxylic acid **(1)** and control molecule 1-[5-(trifluoromethyl)1,3,4-thiadiazol-2-yl]piperazine **(2)** recorded with proton decoupling during the acquisition period in the presence of different concentrations of HSA are shown in Figure 6. ITC measurements performed with **(2)** did not find any evidence of binding to HSA (only heat of dilution was detected with 8 μL injections of 800 μM of **(2)** into 30 μM HSA) in agreement with the NMR results. A concentration of only 25 μM for both molecules was used for the NMR experiments. The low concentration of the reference molecule avoids problems arising from non-specific binding and aggregation. Disadvantages with these molecules are represented by the rapid rotation of the fluorine atoms about the $C_3$ axis of the group observed even in the bound state. This results in a limited difference in linewidth for the $CF_3$ signal of the reference molecule between the free and bound state.

*Molecules with a CF group*

Molecules with a CF group are particularly suited for the competition ligand based screening experiments. The $^{19}F$ CSA can be very large therefore increasing the difference in linewidth between the free and bound state of the reference molecule according to equation (3). For example the CSA for an aromatic CF ranges from 71 ppm for monofluoro-benzene to 158 ppm for hexafluoro-benzene (H. Raber et al., Chem. Phys., 26, 123-130 (1977)). In addition, the $^{19}F$ CSA of the reference molecule in the bound state can increase due to an "ortho effect" or from the direct involvement of the fluorine atom in an hydrogen bond with the protein. These two phenomena also have the effect of rendering a large difference in the isotropical chemical shift for the free and bound state. For a weak affinity reference compound the exchange term of equation (6) can contribute significantly to the linewidth of the reference compound in the presence of the protein. The fluorine signal is usually scalar coupled with several protons and therefore for sensitivity improvement it is necessary to record the spectra with proton decoupling during acquisition. Figure 7 shows typical spin-echo fluorine spectra for the reference molecule 2-hydroxy 3-fluorobenzoic acid **(3)** and control molecule **(2)** recorded with proton decoupling as a function of HSA concentration. A drawback with these molecules is the required higher

concentration for the experiments. The spectra of Figure 7 were recorded with a concentration for the reference molecule of 50 $\mu$M.

*Titration and Screening Experiments*

After the selection of the reference and control molecules, titration experiments as a function of the protein are recorded as shown in Figures 6 and 7. The intensity ratio of the two fluorine signals is plotted as a function of the fraction of protein-bound reference molecule as shown in the example of Figure 8. The fraction of bound compound is calculated by using the dissociation binding constant derived from other techniques (e.g., ITC or fluorescence spectroscopy) as described in (C. Dalvit et al., J. Am. Chem. Soc., 124, 7702-7709 (2002); and C. Dalvit et al., Comb. Chem. HTS, 5, 645-650 (2002)). These techniques also provide the number of binding sites (n) for the reference molecule a parameter that is very important for the competition binding experiments. ITC measurements provided a n value that is close to 4 for **(1)** and close to 1 for **(3)**. Therefore, although molecule **(1)** can still be used for screening purposes with some limitations in the interpretation of the experimental results, it cannot be used for deriving the binding constants of the NMR-hits. Molecule **(3)** represents a suitable reference molecule due to the presence of only one binding site on HSA at the concentration used in these experiments. According to its chemical structure, an aspirin analogue, its putative binding site would be the Sudlow site I (located in subdomain IIA) (T. Peters, Jr., All about Albumin Biochemistry, Genetics, and Medical Applications Academic Press, San Diego, U.S.A. 1996). Two different values for the fraction of protein bound reference molecule **(3)** are extracted in Figure 8 using the two limit values of $K_D$ determined by the experimental error. In this specific case, the ITC derived $K_D$ for **(3)** was 41 $\pm$ 3.3 $\mu$M and thus the two limit values of $K_D$ correspond to 37.7 and 44.3 $\mu$M, respectively.

It should be pointed out that the NMR experiments could also be recorded in the presence of only the reference molecule (C. Dalvit et al., Comb. Chem. HTS, 5, 605-611 (2002) and above in Example (I)). In this case, however, two experiments have to be recorded: one without the CPMG sequence (i.e. $2n\tau = 0$) and another with a CPMG with a long $2n\tau$. The signal intensity ratio extracted from these two spectra is then plotted as a function of the fraction of bound reference molecule.

The graphs of Figure 8 are then used for setting up the experimental conditions necessary for screening. Figure 9 shows the screening process performed against HSA with **(3)** as reference molecule. For screening, a total spin-echo period ($2n\tau$) was selected for which the signal of the reference molecule is approaching zero. The presence in the mixture of 5-$CH_3$ D,L Trp and sucrose (3rd spectra from left), known as non-binders, do not alter the spectrum of the spy molecule. In contrast, the presence in the mixture of the warfarin derivative 4-hydroxy-3-[1-(p-iodophenyl)-3-oxobutyl] coumarin **(4)** (right) results in the reappearance of the signal of **(3)**. This results, according to the graphs of Figure 8, from the displacement of the reference compound from the protein. The extent of displacement can then be used to calculate the binding constant of the NMR-hit (C. Dalvit et al., J. Am. Chem. Soc., 124, 7702-7709 (2002); and C. Dalvit et al., Comb. Chem. HTS, 5, 645-650 (2002)) as described in Table 2.

Table 2. Single-Point NMR-derived binding constant for **(4)** and its comparison with measured fluorescence value

| I**(3)**/I**(2)** | $K_D$ | [I] | [$L_{TOT}$] | [$E_{TOT}$] | [EL] / [$L_{TOT}$] | [EL] | $K_D^{app}$ | $K_I^{NMR}$ | $K_I^{fluo}$ |
|---|---|---|---|---|---|---|---|---|---|
| | | | | | | | | | |
| 0.698 | 44.3 | 25 | 50 | 0.6 | 0.00159 | 0.080 | 326.8 | 3.9 | 3.3 +/- 0.3 |
| 0.698 | 37.7 | 25 | 50 | 0.6 | 0.00171 | 0.086 | 300.4 | 3.6 | |

All the values for the concentration and binding constants are expressed in $\mu$M. $K_D$ is the binding constant of **(3)** derived from ITC and $K_I^{fluo}$ is the binding constant for **(4)** derived from fluorescence. [I], [$L_{TOT}$] and [$E_{TOT}$] are the concentration of **(4)**, **(3)** and HSA, respectively. The $K_I^{NMR}$ is the binding constant for **(4)** derived from the NMR measurements.

In order to derive a reliable value for the binding constant, one also has to record the proton spectrum. This is necessary for calculating the concentration of the NMR-hit by simply comparing the integral of a signal of the reference molecule for which the concentration is known with the integral of a signal of the NMR-hit. The NMR-derived $K_D$ for **(4)** compares favorably with the value derived from a full titration fluorescence measurement. Since its first application (C. Dalvit et al., J. Am. Chem. Soc., 124, 7702-7709 (2002); and C. Dalvit et al., Comb. Chem. HTS, 5, 645-650 (2002)) binding constants have been calculated using this approach for several hundred compounds. For a pure competition binding mechanism and a single binding site, excellent agreement was observed between the single-point NMR derived binding constants and the full titration fluorescence- and ITC- derived binding constants. This NMR approach also allows the determination of high-affinity binding constants that would not be easily obtained with other NMR methods.

*Limit of Detection*

Owing to the large CSA and the large exchange contribution for a weak binding affinity reference molecule it is possible to significantly reduce the concentration of protein needed for screening. This can be appreciated in Figure 10 where the screening is performed with **(3)** in the presence of HSA at a concentration of only 150 nM. Despite the large ratio $[L_{TOT}]/[E_{TOT}]$ (=330) and the small ratio $[EL]/[L_{TOT}]$ (=0.00165 using the $K_D$ of 41 $\mu$M) it is possible to observe the effect of the small fraction of bound ligand and perform the screening at such low protein concentration. This probably represents a fortunate case. According to solid state NMR work, the presence of an OH group in the ortho position to the fluorine atom is responsible for a shift of 50 ppm in the component of the $^{19}F$ chemical shift tensor perpendicular to the aromatic ring resulting in a large $^{19}F$ CSA (H. Raber et al., Chem. Phys., 26, 123-130 (1977)). However, similar behavior was observed with other proteins and with reference molecules containing a para-fluoro benzyl moiety (data not shown). Therefore it is likely that the exchange term also contributes significantly to the observed transverse relaxation. The spectra reported in Figure 10 were recorded with 128 scans and a total measuring time of 10 minutes. The use of cryoprobe technology optimized for $^{19}F$ detection could further improve the detection limits. Protein concentrations as low as 50 to 100 nM could then be used. This will allow screening of a large number of chemical mixtures against proteins that cannot be expressed in high amount (e.g., membrane proteins). Fluorine spectra can be recorded very rapidly with cryoprobe technology. A conservative estimate of a two-fold sensitivity improvement with cryoprobe technology would translate into a four-fold reduction in acquisition time. Therefore the spectra of Figure 10 could have been recorded in just 150 s, thus enhancing the throughput of this screening process. It should be pointed out that problems of radiation damping encountered in proton detected experiments recorded with cryoprobes are absent in the fluorine detected experiments because of the low concentration of the spy and control molecules.

*Screening in the presence of protonated solvents and detergents.*

A particular advantage of the $^{19}F$ ligand-based competition binding experiments is the possibility to perform the screening even in the presence of protonated solvents, buffers, or detergents. The proton spectrum of HSA in the presence of 100 mM HEPES and 1% glycerol is shown in Figure 11. The intense signals of the buffer and glycerol mask the observation of the weak signals of the reference and control molecules necessary for performing the screening. These problems are not encountered in the $^{19}F$ detection experiments. Therefore it is possible to perform the screening as shown in Figure 11 even in these difficult experimental conditions. Because of these properties, fluorine ligand-based competition binding screening experiments are particularly advantageous to the screening of molecules against membrane proteins dissolved in SDS or other detergents. Once a suitable reference molecule has been identified, the $^{19}F$ ligand-based competition binding NMR screening will provide reliable hits. The molecules that simply bind to the membranes and detergents and that appear as potential ligands in different assays will not be detected in the $^{19}F$ experiments described here. Only molecules that compete with the reference molecule are identified. Finally, the experiments can also be used for screening of plant and fungi extracts, and for screening of molecules within living cells.

CONCLUSION

Thus, the use of a weak affinity ligand containing a $^{19}F$ atom in combination with the competition binding experiments permit rapid screening of large chemical mixtures against protein, DNA or RNA fragments. In addition, the method provides a direct determination of the binding constant of the identified NMR-hits. With the $^{19}F$ competition binding HTS experiments there are no problems of overlap and, because of the high sensitivity of $^{19}F$ nucleus (0.83 times the $^1H$ sensitivity), it is possible to rapidly screen large chemical libraries or natural product extracts. In addition, the experiments can be performed for protein solutions in the presence of high concentrations of non-deuterated detergents therefore allowing HTS with membrane proteins. Furthermore, since resonances from the actual molecules screened are not utilized, only the spy and control molecules are required to contain a fluoro moiety. Thus, fluorine-based competition screening should find numerous uses in the pharmaceutical industry, and should further extend the impact of NMR-based screening on the drug discovery process.

The method is rapid and requires only a limited amount of protein and therefore compares favorably with the other established non-NMR techniques used in high-throughput screening. In addition, the method provides within a single experiment a meaningful value for the binding constant of the NMR-hit. The absence of overlap permits screening of large chemical mixtures originating from combinatorial chemistry, medicinal chemistry or natural product extraction. Screening against membrane proteins dissolved in different detergents is also possible with this approach. Finally, it is envisioned that these experiments can be extended to the screening of molecules against a receptor located within living cells. Various modifications and alterations to this invention will become apparent to those skilled in the art without departing from the scope and spirit of this invention. It should be understood that this invention is not intended to be unduly limited by the illustrative embodiments and examples set forth herein. Such examples and embodiments are

presented by way of example only with the scope of the invention intended to be limited only by the claims set forth herein as follows.

## Claims

1. A method of identifying a ligand to a target molecule, the method comprising :

    providing an $^{19}$F-labelled reference compound that interacts with the target molecule ;
    collecting a 1D $^{19}$F nuclear magnetic resonance spectrum of the $^{19}$F- labelled reference compound in the presence of the target molecule;
    providing a test sample comprising at least one test compound;
    collecting a 1D $^{19}$F nuclear magnetic resonance spectrum of the $^{19}$F- labelled reference compound in the presence of each test sample and the target molecule ;
    comparing the spectrum of the $^{19}$F-labelled reference compound in the presence of the target molecule to the spectrum of the $^{19}$F-labelled reference compound in the presence of each test sample and the target molecule to determine a change in one or more of the $^{19}$F-labelled reference compound resonances; and
    identifying at least one test compound that interacts with the target molecule, wherein the test compound displaces the $^{19}$F-labelled reference compound.

2. The method of claim 1 wherein the test compound has a binding affinity at least as tight as that of the reference compound.

3. The method of claim 1 wherein a change in one or more of the reference compound resonances comprises an increase in signal intensity in at least one reference resonance.

4. The method of claim 1 wherein identifying at least one test compound comprises recording separate 1 D $^{19}$F nuclear magnetic resonance spectra of the $^{19}$F-labelled reference compound in the presence of each test compound and the target molecule.

5. The method of claim 1 further comprising:

    collecting 1 D $^{19}$F nuclear magnetic resonance spectra of the reference compound in the presence of the target molecule at different concentrations of the $^{19}$F-labelled reference compound; and
    determining the dissociation constant of the test compound.

6. The method of claim 1 further comprising:

    collecting 1D $^{19}$F nuclear magnetic resonance spectra of the $^{19}$F-labelled reference compound in the presence of the target molecule at different concentrations of the target molecule; and
    determining the dissociation constant of the test compound.

7. The method of claim 1 wherein prior to collecting a 1D $^{19}$F nuclear magnetic resonance spectrum of the $^{19}$F-labelled reference compound in the presence of the target molecule for use in the comparing step, the method comprises :

    collecting 1D $^{19}$F nuclear magnetic resonance spectra of the 19F-labelled reference compound in the presence of the target molecule at different concentrations of the target molecule or at different concentrations of the $^{19}$F-labelled reference compound; and
    determining the optimum experimental conditions for identifying at least one test compound that interacts with the target molecule.

8. The method of claim 1 wherein the target molecule is a macromolecule.

9. The method of claim 8 wherein the macromolecule is a polypeptide or polynucleotide.

10. The method of claim 8 wherein the macromolecule is a protein.

11. The method of claim 1 wherein the reference compound binds to the target molecule with a binding affinity in the

micromolar range.

**12.** The method of claim 11 wherein the binding affinity of the reference compound is determined by isothermal titration calorimetry or fluorescence spectroscopy.

**13.** The method of claim 1 further comprising a step of identifying the reference compound comprising:

collecting a WaterLOGSY nuclear magnetic resonance spectrum of a potential reference compound in the absence of the target molecule;
collecting a WaterLOGSY nuclear magnetic resonance spectrum of the potential reference compound in the presence of the target molecule; and
comparing the WaterLOGSY spectra to identify whether the potential reference compound interacts with the target molecule.

**14.** The method of claim 1 wherein the test sample comprises a mixture of two or more test compounds.

**15.** The method of claim 14 further comprising :

collecting a 1D $^{19}F$ nuclear magnetic resonance spectrum of the $^{19}F$- labelled reference compound in the presence of each test compound and the target molecule; and
comparing the spectrum of the reference compound in the presence of the target molecule to the spectrum of the reference compound in the presence of each test compound and the target molecule to determine a change in the selected $^{19}F$-labelled reference compound resonance.

**16.** The method of claim 1 wherein the test compound has a binding affinity tighter than that of the reference compound.

**17.** The method of claim 1 wherein:

providing an $^{19}F$-labelled reference compound comprises providing an $^{19}F$-labelled reference compound and an ERETIC signal with defined linewidth, amplitude, and frequency;
collecting a 1D $^{19}F$ nuclear magnetic resonance spectrum of the $^{19}F$- labelled reference compound in the presence of the target molecule comprises collecting a spectrum of the $^{19}F$-labelled reference compound with the ERETIC signal in the presence of the target molecule; and
collecting a 1D $^{19}F$ nuclear magnetic resonance spectrum of the $^{19}F$- labelled reference compound in the presence of each test sample and the target molecule comprises collecting a spectrum of the $^{19}F$-labelled reference compound with the ERETIC signal in the presence of each test sample and the target molecule.

**18.** The method of claim 1 wherein:

providing an $^{19}F$-labelled reference compound comprises providing an $^{19}F$-labelled reference compound and an $^{19}F$-labelled non-interacting compound;
collecting a 1 D $^{19}F$ nuclear magnetic resonance spectrum of the $^{19}F$- labelled reference compound in the presence of the target molecule comprises collecting a spectrum of the $^{15}F$-labelled reference compound and the $^{19}F$- labelled non-interacting compound in the presence of the target molecule; and
collecting a 1D $^{19}F$ nuclear magnetic resonance spectrum of the $^{19}F$- labelled reference compound in the presence of each test sample and the target molecule comprises collecting a spectrum of the $^{19}F$-labelled reference compound and the $^{19}F$-labelled non-interacting compound in the presence of each test sample and the target molecule.

**19.** The method of claim 1 wherein providing a test sample comprises providing a plurality of test samples, each test sample comprising at least one test compound.

**Patentansprüche**

**1.** Verfahren zum Identifizieren eines Liganden an einem Zielmolekül, wobei das Verfahren umfasst:

Bereitstellen einer $^{19}F$-markierten Referenzverbindung, die mit dem Zielmolekül in Wechselwirkung tritt;

Erfassen eines eindimensionalen (1D) magnetischen $^{19}F$-Kernresonanzspektrums von der $^{19}F$-markierten Referenzverbindung in Anwesenheit von dem Zielmolekül;
Bereitstellen einer Untersuchungsprobe, die mindestens eine Testverbindung umfasst;
Erfassen eines eindimensionalen (1D) magnetischen $^{19}F$-Kernresonanzspektrums von der $^{19}F$-markierten Referenzverbindung in Anwesenheit von jeder Untersuchungsprobe und von dem Zielmolekül;
Vergleichen des Spektrums von der $^{19}F$-markierten Referenzverbindung in Anwesenheit von dem Zielmolekül mit dem Spektrum von der $^{19}F$-markierten Referenzverbindung in Anwesenheit von jeder Untersuchungsprobe und dem Zielmolekül zur Bestimmung einer Veränderung in einer oder mehreren Resonanzen der $^{19}F$-markierten Referenzverbindung; und
Identifizieren von mindestens einer Testverbindung, die mit dem Zielmolekül in Wechselwirkung tritt, wobei die Testverbindung die $^{19}F$-markierte Referenzverbindung verdrängt.

**2.** Verfahren nach Anspruch 1, wobei die Testverbindung eine Bindungsaffinität aufweist, die mindestens so stark ist wie diejenige der Referenzverbindung.

**3.** Verfahren nach Anspruch 1, wobei eine veränderung bei einer oder mehreren Resonanzen der Referenzverbindung eine Zunahme der Signalintensität bei mindestens einer Resonanz der Referenzverbindung umfasst.

**4.** Verfahren nach Anspruch 1, wobei das Identifizieren von mindestens einer Testverbindung das Aufzeichnen von separaten, eindimensionalen (1D) magnetischen $^{19}F$-Kernresonanzspektren der $^{19}F$-markierten Referenzverbindung in Anwesenheit jeder einzelnen Testverbindung und des Zielmoleküls umfasst.

**5.** Verfahren nach Anspruch 1, das ferner umfasst:

Erfassen von eindimensionalen (1D) magnetischen $^{19}F$-Kernresonanzspektren der Referenzverbindung in Anwesenheit von dem Zielmolekül bei unterschiedlichen Konzentrationen von der $^{19}F$-markierten Referenzverbindung; und
Bestimmen der Dissoziationskonstante der Testverbindung.

**6.** Verfahren nach Anspruch 1, das ferner umfasst:

Erfassen von eindimensionalen (1D) magnetischen $^{19}F$-Kernresonanzspektren der $^{19}F$-markierten Referenzverbindung in Anwesenheit des Zielmoleküls bei unterschiedlichen Konzentrationen von dem Zielmolekül; und
Bestimmen der Dissoziationskonstante der Testverbindung.

**7.** Verfahren nach Anspruch 1, wobei vor dem Erfassen eines eindimensionalen (1D) magnetischen $^{19}F$-Kernresonanzspektrums von der $^{19}F$-markierten Referenzverbindung in Anwesenheit von dem Zielmolekül zur Verwendung in dem Vergleichsschritt das Verfahren umfasst:

Erfassen von eindimensionalen (1D) magnetischen $^{19}F$-Kernresonanzspektren der $^{19}F$-markierten Referenzverbindung in Anwesenheit des Zielmoleküls bei unterschiedlichen Konzentrationen von dem Zielmolekül oder unterschiedlichen Konzentrationen von der $^{19}F$-markierten Referenzverbindung; und
Bestimmen der optimalen experimentellen Bedingungen zur Identifizierung von mindestens einer Testverbindung, die mit dem Zielmolekül in Wechselwirkung tritt.

**8.** Verfahren nach Anspruch 1, wobei das Zielmolekül ein Makromolekül ist.

**9.** Verfahren nach Anspruch 8, wobei das Makromolekül ein Polypeptid oder ein Polynukleotid ist.

**10.** Verfahren nach Anspruch 8, wobei das Makromolekül ein Protein ist.

**11.** Verfahren nach Anspruch 1, wobei die Referenzverbindung mit einer Bindungsaffinität im mikromolaren Bereich an das Zielmolekül bindet.

**12.** Verfahren nach Anspruch 11, wobei die Bindungsaffinität von der Referenzverbindung durch isothermale Titrationskalorimetrie oder durch Fluoreszenzspektroskopie bestimmt wird.

**13.** Verfahren nach Anspruch 1, ferner enthaltend einen Schritt zum Identifizieren der Referenzverbindung, der umfasst:

Erfassen eines magnetischen Kernresonanzspektrums von einer potentiellen Referenzverbindung in Abwesenheit von dem Zielmolekül mit dem WaterLOGSY-Verfahren;
Erfassen eines magnetischen Kernresonanzspektrums von einer potentiellen Referenzverbindung in Anwesenheit von dem Zielmolekül mit dem WaterLOGSY-Verfahren; und
Vergleichen der mit dem WaterLOGSY-Verfahren erhaltenen Spektren, um zu ermitteln, ob die potentielle Referenzverbindung mit dem Zielmolekül in Wechselwirkung tritt.

**14.** Verfahren nach Anspruch 1, wobei die Untersuchungsprobe eine Mischung aus zwei oder mehr Testverbindungen umfasst.

**15.** Verfahren nach Anspruch 14, das ferner umfasst:

Erfassen eines eindimensionalen (1D) magnetischen $^{19}$F-Kernresonanzspektrums von der $^{19}$F-markierten Referenzverbindung in Anwesenheit von jeder Untersuchungsprobe und dem Zielmolekül; und
Vergleichen des Spektrums von der Referenzverbindung in Anwesenheit von dem Zielmolekül mit dem Spektrum von der Referenzverbindung in Anwesenheit von jeder Testverbindung und dem Zielmolekül zur Bestimmung einer Veränderung der Resonanz in der ausgewählten $^{19}$F-markierten Referenzverbindung.

**16.** Verfahren nach Anspruch 1, wobei die Testverbindung eine Bindungsaffinität aufweist, die stärker ist als diejenige von der Referenzverbindung.

**17.** Verfahren nach Anspruch 1, wobei:

das Bereitstellen einer $^{19}$F-markierten Referenzverbindung das zur Verfügung stellen einer $^{19}$F-markierten Referenzverbindung und eines externen elektrischen Referenzsignals (ERETIC-Signal) mit einer definierten Linienbreite, Amplitude und Frequenz umfasst;
das Erfassen eines eindimensionalen (1D) magnetischen $^{19}$F-Kernresonanzspektrums von der $^{19}$F-markierten Referenzverbindung in Anwesenheit von dem Zielmolekül das Aufzeichnen eines Spektrums von der $^{19}$F-markierten Referenzverbindung mit dem ERETIC-Signal in Anwesenheit von dem Zielmolekül umfasst; und
das Erfassen eines eindimensionalen (1D) magnetischen $^{19}$F-Kernresonanzspektrums von der $^{19}$F-markierten Referenzverbindung in Anwesenheit von dem Zielmolekül das Aufzeichnen eines Spektrums von der $^{19}$F-markierten Referenzverbindung mit dem ERETIC-Signal in Anwesenheit von jeder Untersuchungsprobe und dem Zielmolekül umfasst.

**18.** Verfahren nach Anspruch 1, wobei:

das Bereitstellen einer $^{19}$F-markierten Referenzverbindung das zur Verfügung stellen einer $^{19}$F-markierten Referenzverbindung und einer mit $^{19}$F-markierten, nicht in Wechselwirkung tretenden Verbindung umfasst;
das Erfassen eines eindimensionalen (1D) magnetischen $^{19}$F-Kernresonanzspektrums von der $^{19}$F-markierten Referenzverbindung das Aufzeichnen eines Spektrums von der $^{19}$F-markierten Referenzverbindung und von der mit $^{19}$F-markierten, nicht in Wechselwirkung tretenden Verbindung in Anwesenheit von dem Zielmolekül umfasst; und
das Erfassen eines eindimensionalen (1D) magnetischen $^{19}$F-Kernresonanzspektrums von der $^{19}$F-markierten Referenzverbindung in Anwesenheit von jeder Untersuchungsprobe und von dem Zielmolekül das Aufzeichnen eines Spektrums von der $^{19}$F-markierten Referenzverbindung und von der mit $^{19}$F-markierten, nicht in Wechselwirkung tretenden Verbindung in Anwesenheit von jeder Untersuchungsprobe und dem Zielmolekül umfasst.

**19.** Verfahren nach Anspruch 1, wobei das Bereitstellen einer Untersuchungsprobe das zur Verfügung stellen mehrerer Untersuchungsproben umfasst, wobei jede Untersuchungsprobe mindestens eine Testverbindung umfasst.

## Revendications

**1.** Procédé d'identification d'un ligand à une molécule cible, le procédé comprenant les étapes consistant à :

fournir un composé de référence marqué par $^{19}$F qui interagit avec la molécule cible ;
recueillir un spectre par résonance magnétique nucléaire 1D $^{19}$F du composé de référence marqué par $^{19}$F en présence de la molécule cible ;

fournir un échantillon d'essai comprenant au moins un composé d'essai ;
recueillir un spectre par résonance magnétique nucléaire 1D $^{19}F$ du composé de référence marqué par $^{19}F$ en présence de chaque échantillon d'essai et de molécule cible ;
comparer le spectre du composé de référence marqué par $^{19}F$ en présence de la molécule cible au spectre du composé de référence marqué par $^{19}F$ en présence de chaque échantillon d'essai et de la molécule cible pour déterminer un changement dans une ou plusieurs des résonances du composé de référence marqué par $^{19}F$ ; et
identifier au moins un composé d'essai qui interagit avec la molécule cible, dans lequel le composé d'essai déplace le composé de référence marqué par $^{19}F$.

**2.** Procédé selon la revendication 1, dans lequel le composé d'essai présente une affinité de liaison au moins aussi intime que celle du composé de référence.

**3.** Procédé selon la revendication 1, dans lequel un changement dans une ou plusieurs des résonances du composé de référence comprend une augmentation de l'intensité du signal dans au moins une résonance de référence.

**4.** Procédé selon la revendication 1, dans lequel l'étape consistant à identifier au moins un composé d'essai comprend l'étape consistant à enregistrer des spectres par résonance magnétique nucléaire 1D $^{19}F$ séparés du composé de référence marqué par $^{19}F$ en présence de chaque composé d'essai et de la molécule cible.

**5.** Procédé selon la revendication 1, comprenant en outre les étapes consistant à :

recueillir des spectres par résonance magnétique nucléaire 1D $^{19}F$ du composé de référence en présence de la molécule cible à différentes concentrations du composé de référence marqué par $^{19}F$ ; et
déterminer la constante de dissociation du composé d'essai.

**6.** Procédé selon la revendication 1 comprenant en outre les étapes consistant à :

recueillir des spectres par résonance magnétique nucléaire 1D $^{19}F$ du composé de référence marqué par $^{19}F$ en présence de la molécule cible à différentes concentrations de la molécule cible ; et
déterminer la constante de dissociation du composé d'essai.

**7.** Procédé selon la revendication 1, dans lequel avant l'étape consistant à recueillir un spectre par résonance magnétique nucléaire 1D $^{19}F$ du composé de référence marqué par $^{19}F$ en présence de la molécule cible à utiliser dans l'étape de comparaison, le procédé comprend les étapes consistant à :

recueillir des spectres par résonance magnétique nucléaire 1D $^{19}F$ du composé de référence marqué par $^{19}F$ en présence de la molécule cible à différentes concentrations de la molécule cible ou à différentes concentrations du composé de référence marqué par $^{19}F$ ; et
déterminer les conditions expérimentales optimales pour identifier au moins un composé d'essai qui interagit avec la molécule cible.

**8.** Procédé selon la revendication 1, dans lequel la molécule cible est une macromolécule.

**9.** Procédé selon la revendication 8, dans lequel la macromolécule est un polypeptide ou un polynucléotide.

**10.** Procédé selon la revendication 8, dans lequel la macromolécule est une protéine.

**11.** Procédé selon la revendication 1, dans lequel le composé de référence se lie à la molécule cible avec une affinité de liaison dans la gamme micromolaire.

**12.** Procédé selon la revendication 11, dans lequel l'affinité de liaison du composé de référence est déterminée par titration calorimétrique isotherme ou spectroscopie par fluorescence.

**13.** Procédé selon la revendication 1, comprenant en outre une étape consistant à identifier le composé de référence comprenant les étapes consistant à :

recueillir un spectre par résonance magnétique nucléaire WaterLOGSY d'un composé de référence potentiel en l'absence de la molécule cible ;

recueillir un spectre par résonance magnétique nucléaire WaterLOGSY d'un composé de référence potentiel en présence de la molécule cible ; et
comparer les spectres WaterLOGSY pour identifier si le composé de référence potentiel interagit avec la molécule cible.

**14.** Procédé selon la revendication 1, dans lequel l'échantillon d'essai comprend un mélange de deux composés d'essai ou plus.

**15.** Procédé selon la revendication 14, comprenant en outre les étapes consistant à :

recueillir un spectre par résonance magnétique nucléaire 1D $^{19}F$ du composé de référence marqué par $^{19}F$ en présence de chaque composé d'essai et de la molécule cible ; et
comparer le spectre du composé de référence en présence de la molécule cible au spectre du composé de référence en présence de chaque composé d'essai et de la molécule cible pour déterminer un changement dans la résonance du composé de référence marqué par $^{19}F$ sélectionné.

**16.** Procédé selon la revendication 1, dans lequel le composé d'essai présente une affinité de liaison plus intime que celle du composé de référence.

**17.** Procédé selon la revendication 1, dans lequel :

l'étape consistant à fournir un composé de référence
marqué par $^{19}F$ comprend l'étape consistant à fournir un composé de référence marqué par $^{19}F$ et un signal ERETIC avec une largeur spectrale, une amplitude et
une fréquence définies ;
l'étape consistant à recueillir un spectre par résonance magnétique nucléaire 1D $^{19}F$ du composé de référence marqué par $^{19}F$ en présence de la molécule cible comprend l'étape consistant à recueillir un spectre du composé de référence marqué par $^{19}F$ avec le
signal ERETIC en présence de la molécule cible ; et
l'étape consistant à recueillir un spectre par résonance magnétique nucléaire 1D $^{19}F$ du composé de référence marqué par $^{19}F$ en présence de chaque échantillon d'essai et de la molécule cible comprend l'étape consistant à recueillir un spectre du composé de référence marqué par $^{19}F$ avec le signal ERETIC en présence de chaque échantillon d'essai et de la molécule cible.

**18.** Procédé selon la revendication 1, dans lequel :

l'étape consistant à fournir un composé de référence marqué par $^{19}F$ comprend l'étape consistant à fournir un composé de référence marqué par $^{19}F$ et un composé marqué par $^{19}F$ sans interaction ;
l'étape consistant à recueillir un spectre par résonance magnétique nucléaire 1D $^{19}F$ du composé de référence marqué par $^{19}F$ en présence de la molécule cible comprend l'étape consistant à recueillir un spectre du composé de référence marqué par $^{19}F$ et du composé marqué par $^{19}F$ sans interaction en présence de la molécule cible ; et
l'étape consistant à recueillir un spectre par résonance magnétique nucléaire 1D $^{19}F$ du composé de référence marqué par $^{19}F$ en présence de chaque échantillon d'essai et de la molécule cible comprend l'étape consistant à recueillir un spectre du composé de référence marqué par $^{19}F$ et du composé marqué par $^{19}F$ sans interaction en présence de chaque échantillon d'essai et de la molécule cible.

**19.** Procédé selon la revendication 1, dans lequel l'étape consistant à fournir un échantillon d'essai comprend l'étape consistant à fournir une pluralité d'échantillons d'essai, chaque échantillon d'essai comprenant au moins un composé d'essai.

*Fig. 1*

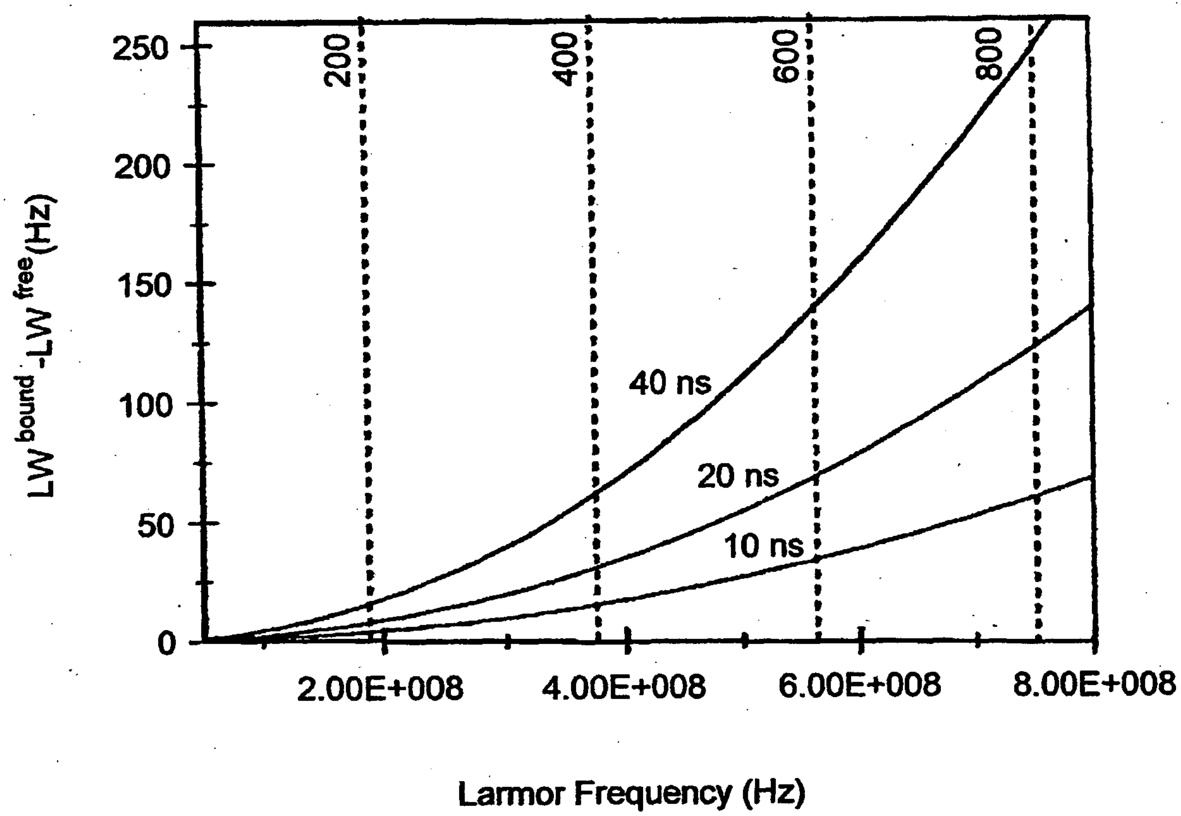
250
200
150
100
50
0
$LW^{bound}-LW^{free}$(Hz)
200
400
600
800
40 ns
20 ns
10 ns
2.00E+008
4.00E+008
6.00E+008
8.00E+008
Larmor Frequency (Hz)

*Fig. 2*

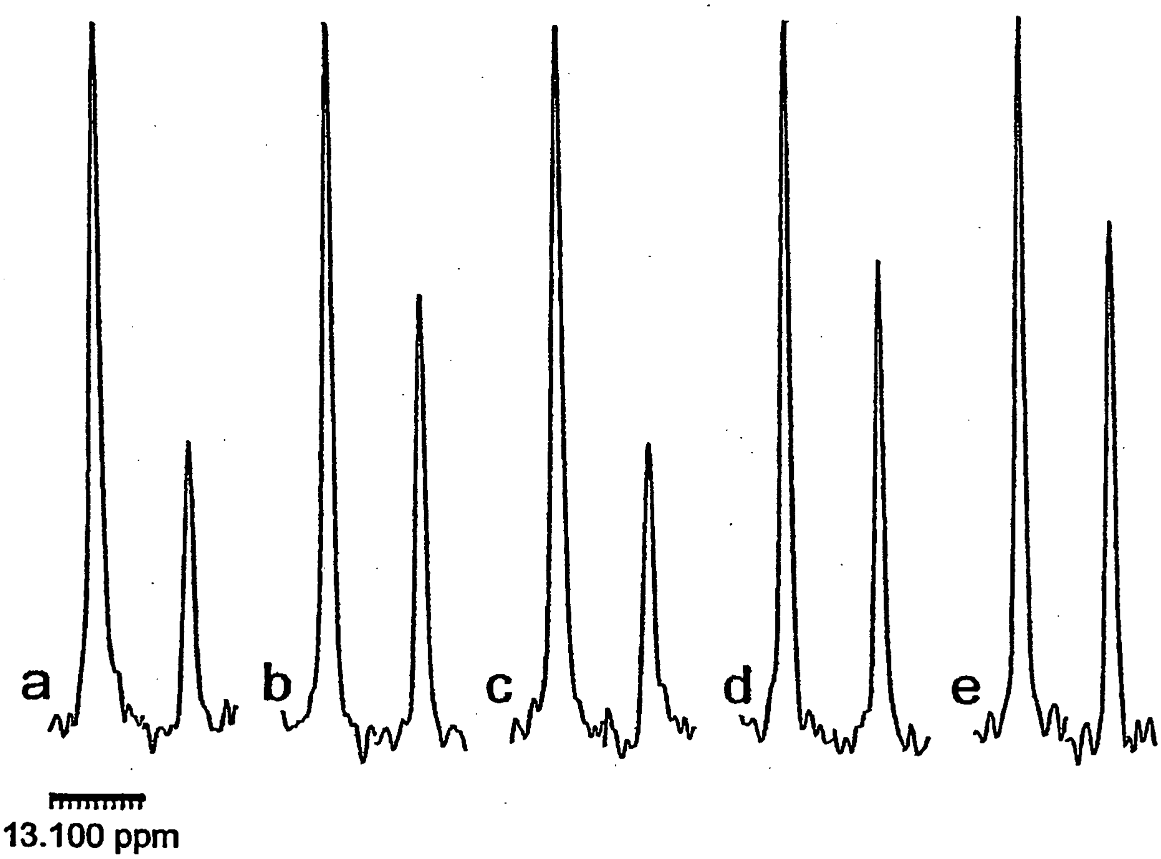

*Fig. 3*

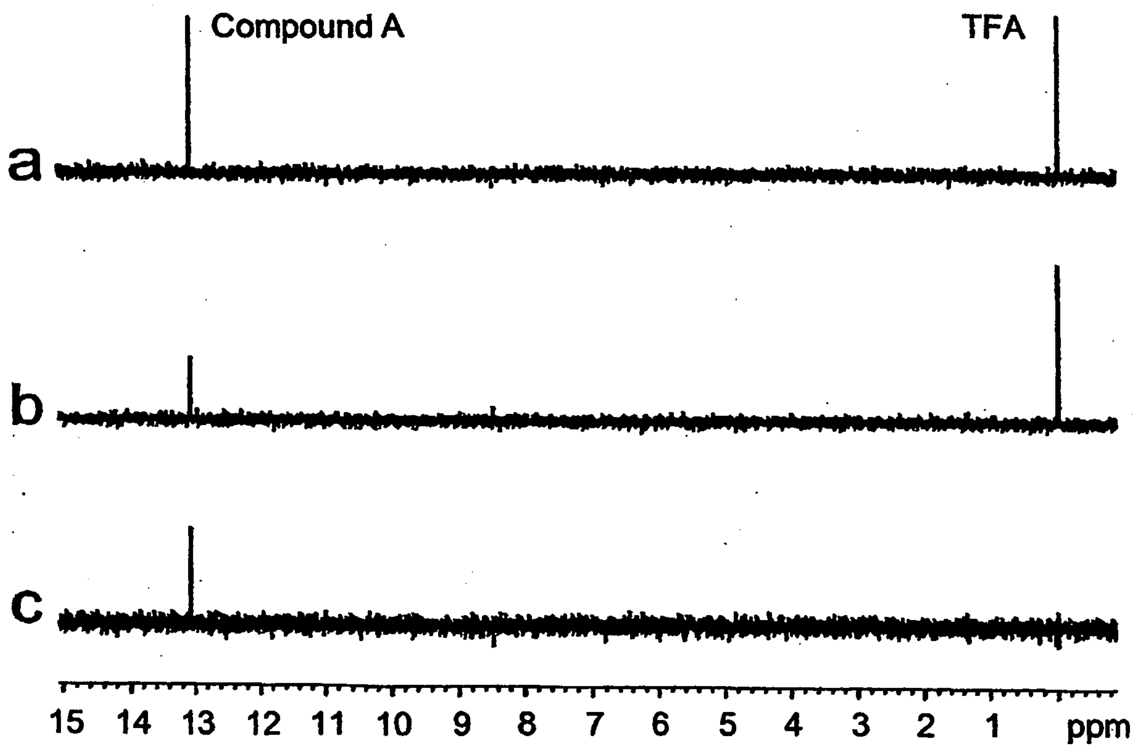

*Fig. 4*

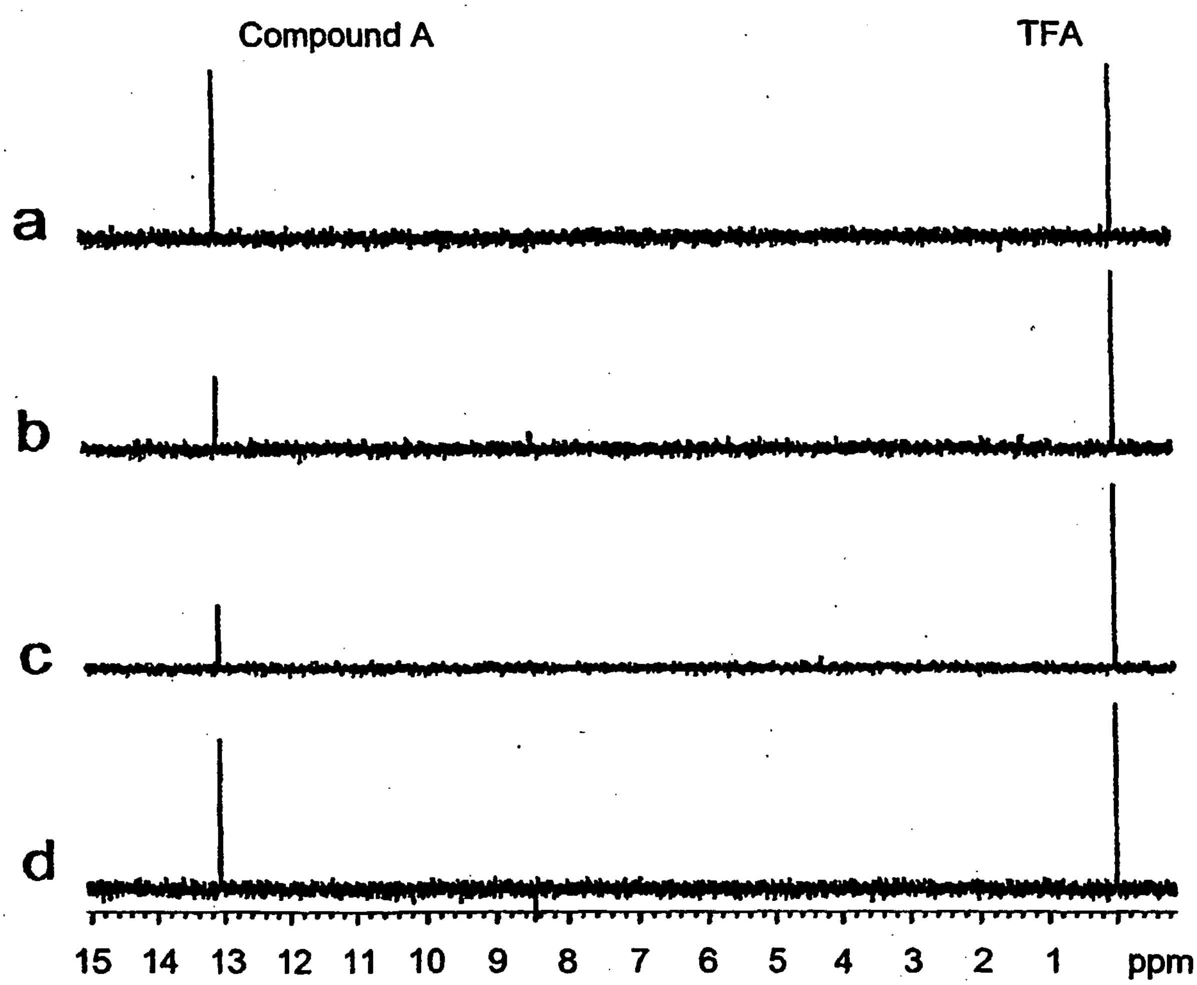

*Fig. 5*

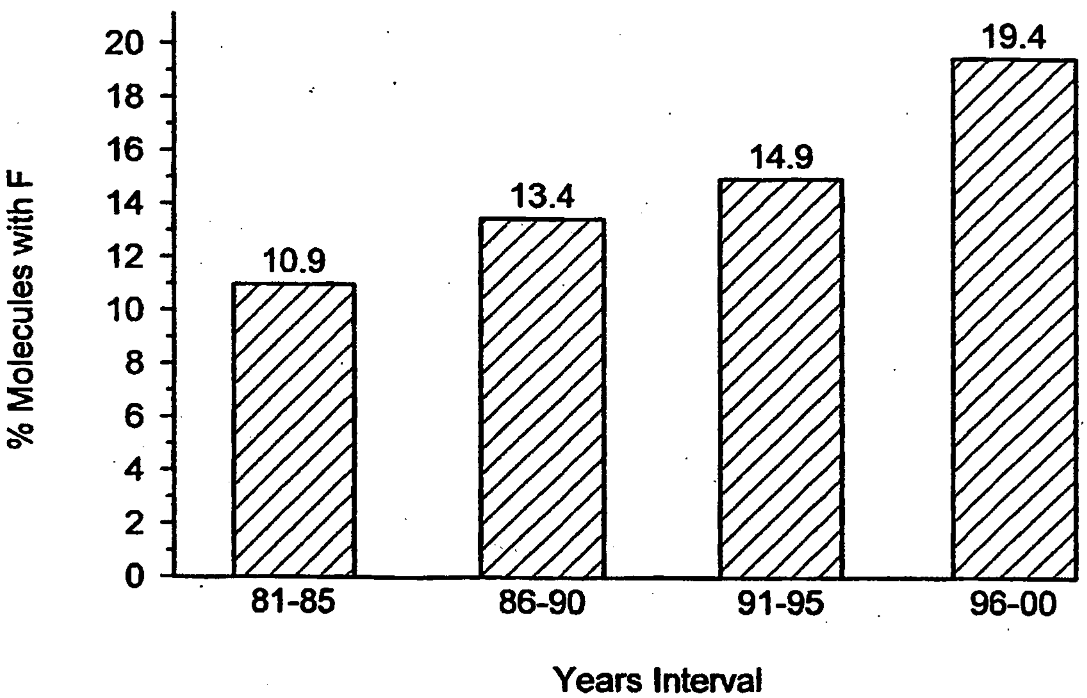
20
18
16
14
12
10
8
6
4
2
0
% Molecules with F
10.9
13.4
14.9
19.4
81-85
86-90
91-95
96-00
Years Interval

*Fig. 6*

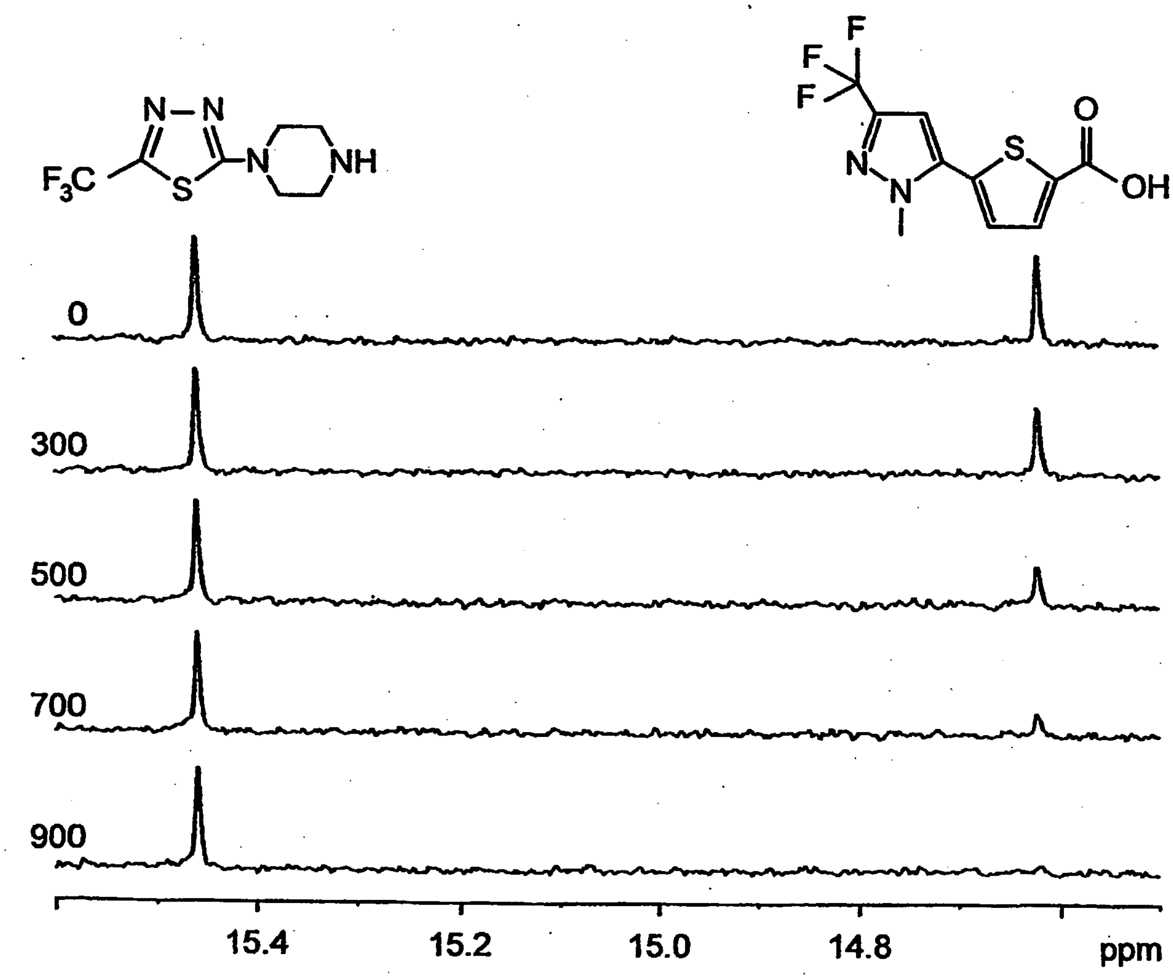

*Fig. 7*

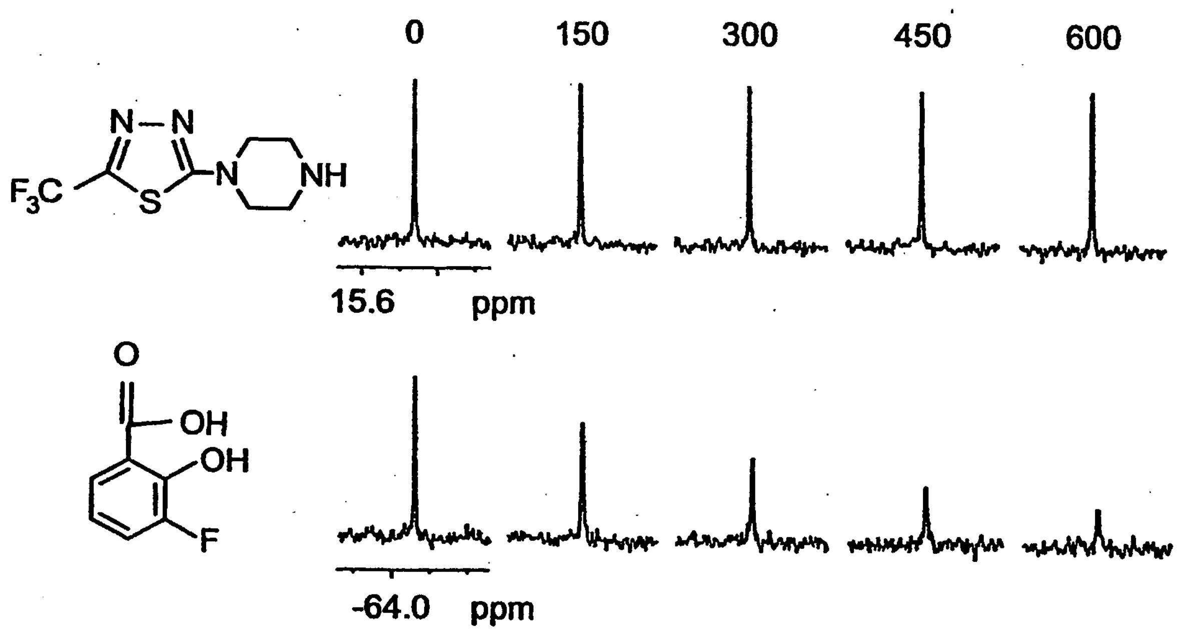

*Fig. 8*

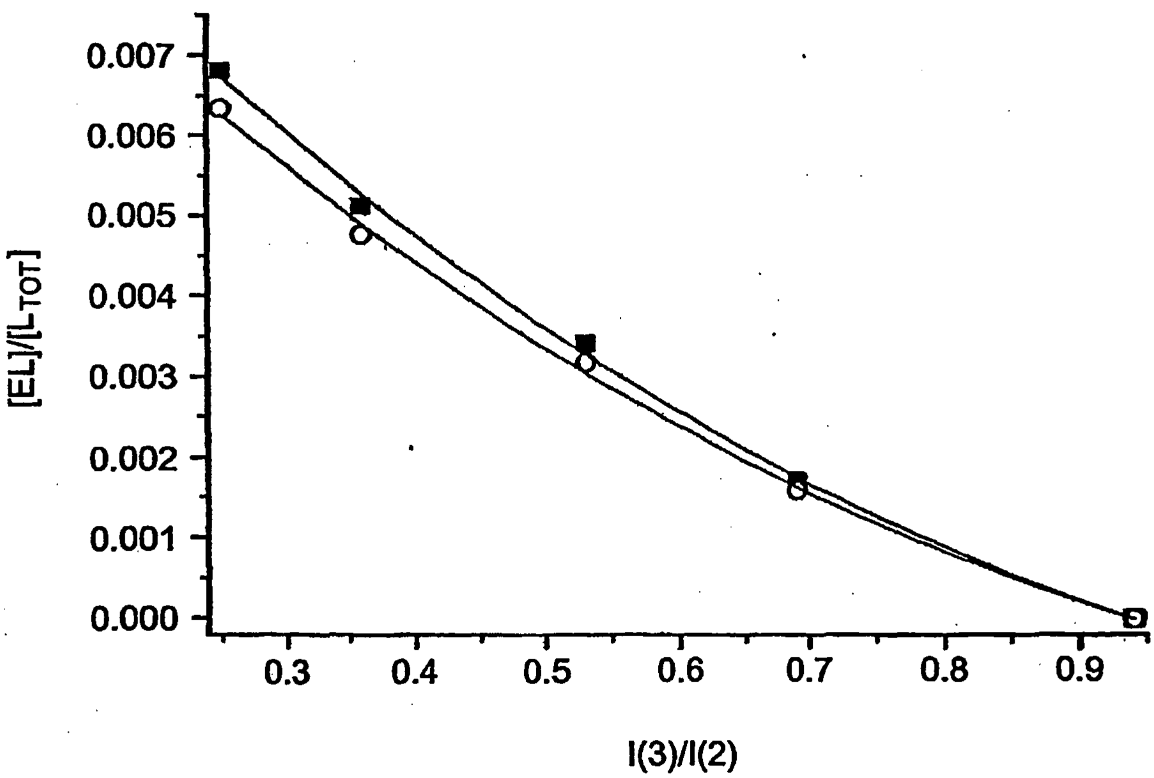
0.007
0.006
0.005
0.004
0.003
0.002
0.001
0.000
[EL]/[L-TOT]
0.3
0.4
0.5
0.6
0.7
0.8
0.9
I(3)/I(2)

*Fig. 9*

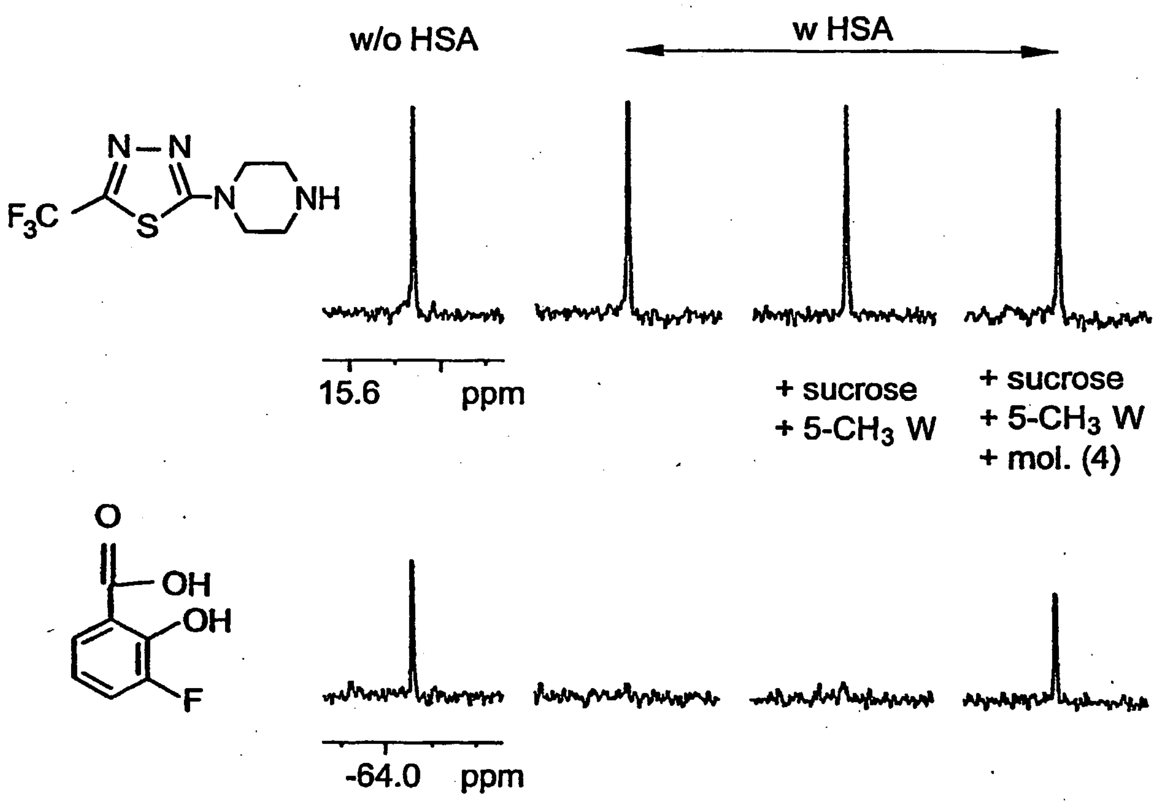

*Fig. 10*

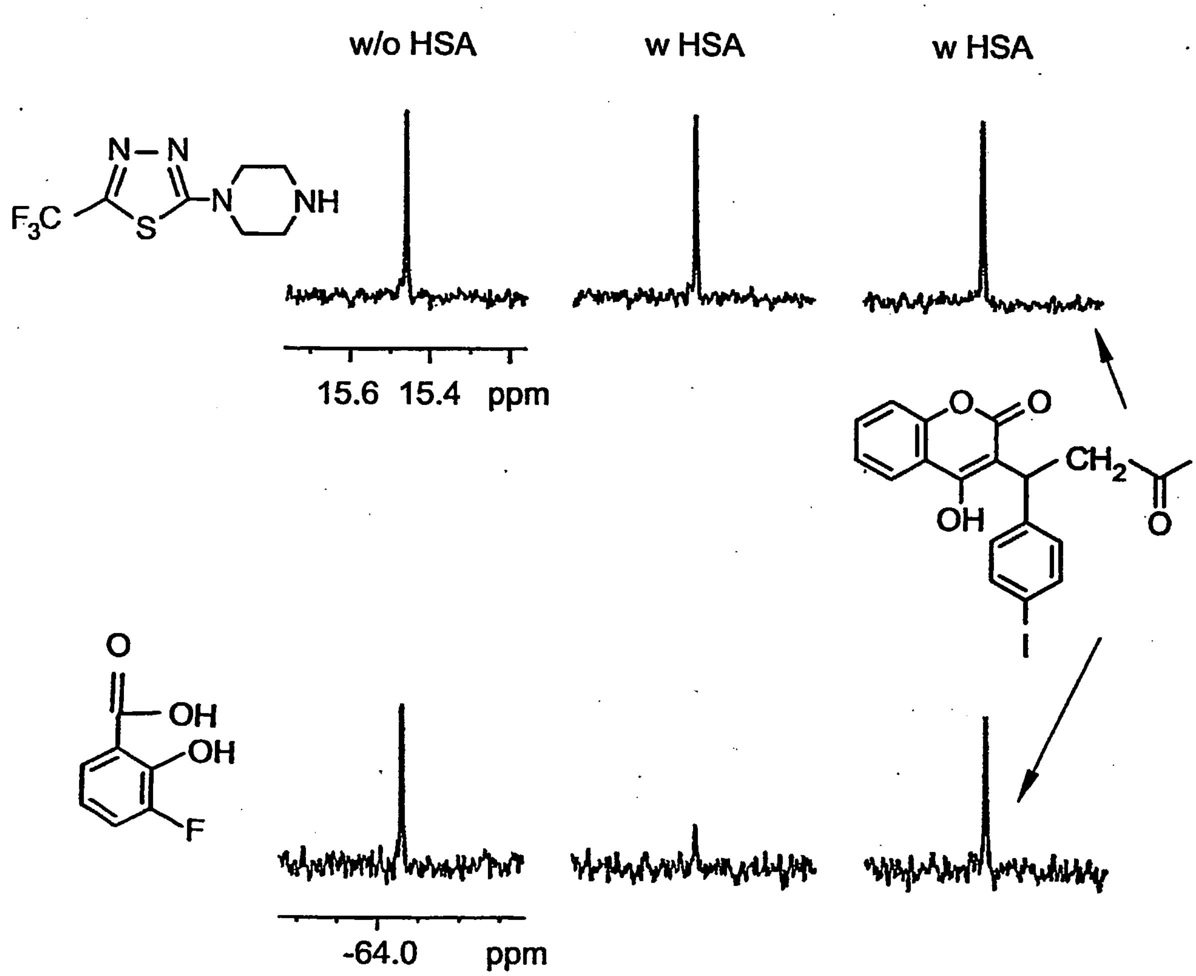

*Fig. 11*

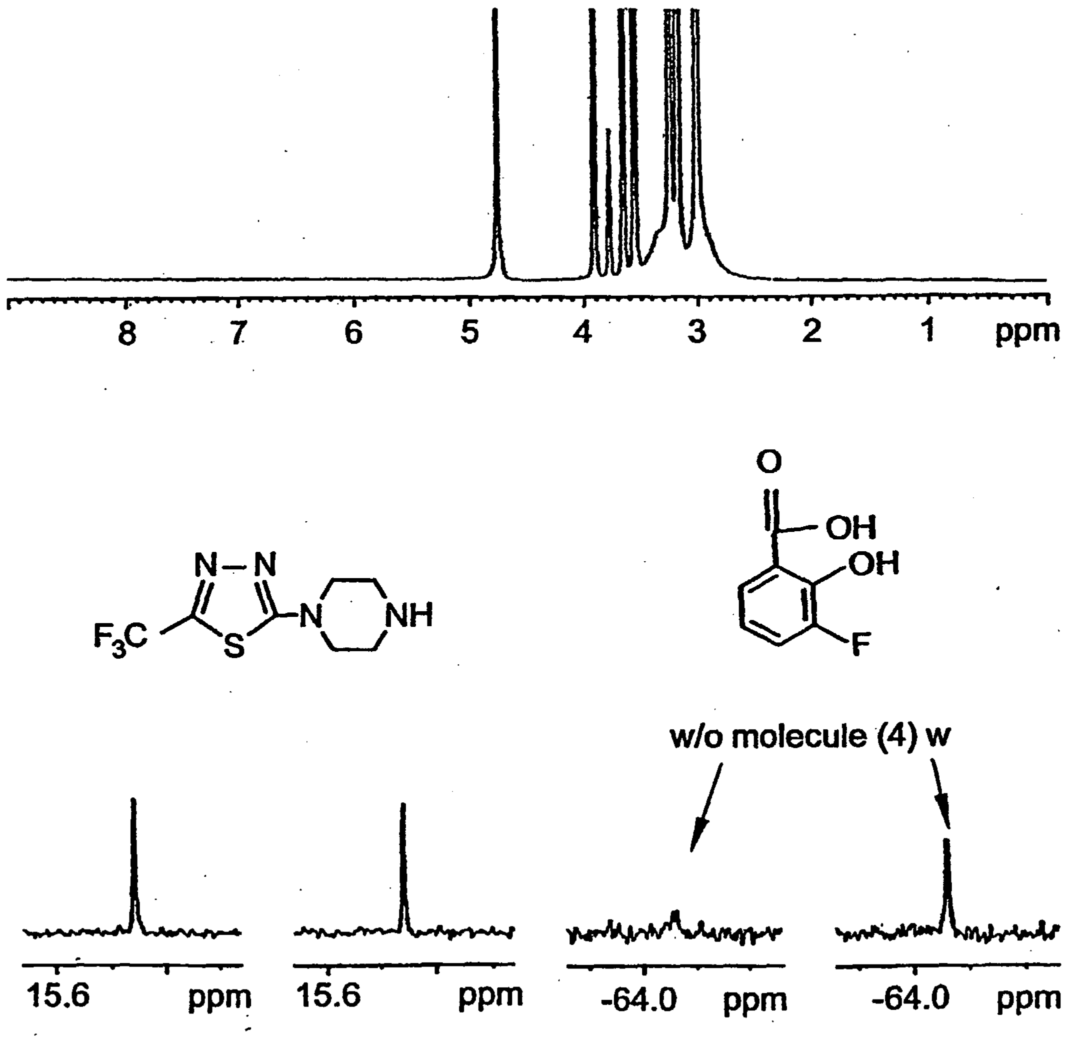

*Fig. 12*

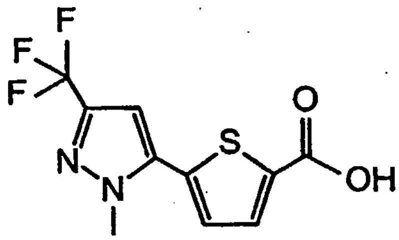

(1)

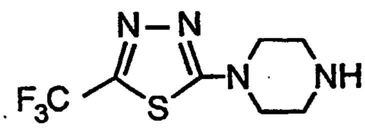

(2)

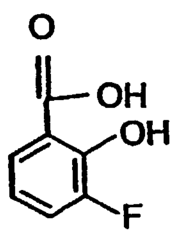

(3)

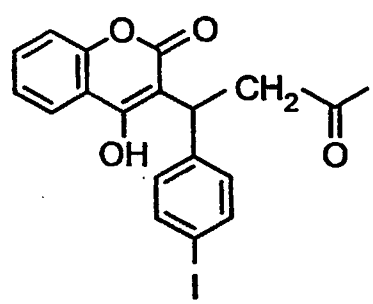

(4)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 0123330 A

### Non-patent literature cited in the description


- **Dalvit C. et al.** NMR-Based Screening with Competition Water-Ligand Observed via Gradient Spectroscopy Experiments: Detection of High-Affinity Ligands. *J. Med. Chem.,* 2002, vol. 45, 2610-2614
- **Jenkins B.G.** Detection of Site-Specific Binding and Co-Binding of Ligands to Macromolecules using 19F NMR. *Life Sciences,* 1991, vol. 48, 1227-1240
- **C. Dalvit et al.** *J. Am. Chem. Soc.,* 2002, vol. 124, 7702-7709
- **S. Akoka et al.** *Anal. Chem.,* 1999, vol. 71, 2554-2557
- **V. Silvestre et al.** *S. Anal, Chem.,* 2001, vol. 73, 1862-1868
- **C. Dalvit et al.** *J. Biomol. NMR,* 2000, vol. 18, 65-68
- **T.-L. Hwang.** *J. Magn. Reson.,* 1995, vol. A (112), 275-279
- **T. Wiseman et al.** *Anal. Biochem.,* 1989, vol. 179, 131-137
- **M. Goldman.** Quantum Description of High-Resolution NMR in Liquids. Clarendon Press, 1988
- **J.T. Gerig.** *Methods in Enzymol.,* 1989, vol. 177, 3-23
- **J.T. Gerig.** *Prog. NMR Spectrosc.,* 1994, vol. 26, 293-370
- **J.W. Peng.** *J. Magn. Reson.,* 2001, vol. 153, 32-47
- **J. Feeney et al.** *J. Am. Chem. Soc.,* 1996, vol. 118, 8700-8706
- **D. Canet.** Nuclear Magnetic Resonance Concepts and Methods. John Wiley & Sons, 1996
- **C. Dalvit et al.** *Comb. Chem. HTS,* 2002, vol. 5, 605-611
- **W.E. Hull et al.** *J. Mol. Biol.,* 1975, vol. 98, 121-153
- **H.Y. Carr et al.** *Phys. Rev.,* 1954, vol. 94, 630-638
- **S. Meiboom et al.** *Rev. Sci. Instrum.,* 1958, vol. 29, 688
- Pulse and Fourier Transform NMR. **T.C. Farrar et al.** Introduction to Theory and Methods. Academic Press, 1971
- **Z. Luz et al.** *J. Chem. Phys.,* 1963, vol. 39, 366-370
- **A. Allerhand et al.** *H.S. J. Chem. Phys.,* 1964, vol. 41, 2115-2126
- **H. Raber et al.** *Chem. Phys.,* 1977, vol. 26, 123-130
- **C. Dalvit et al.** *Comb. Chem. HTS,* 2002, vol. 5, 645-650
- **T. Peters, Jr.** All about Albumin Biochemistry, Genetics, and Medical Applications. Academic Press, 1996